# EUROPEAN PATENT APPLICATION

(11) **EP 3 128 007 A1**
(43) Date of publication of application: **08.02.2017**
(21) Application number: 15180308.7
(22) Date of filing: 07.08.2015
(51) Int. Cl.: C12N 15/113, A61K 31/7125, A61K 9/107

(54) **STERILE EMULSION COMPRISING A STABLE PHOSPHOROTHIOATE OLIGONUCLEOTIDE**

(71) Applicant: Gene Signal International SA, 1015 Lausanne (CH)
(72) Inventor: AL-MAHMOOD, Salman, 75014 Paris (FR); COLIN, Sylvie, 94230 Cachan (FR); BONGAERTS, Maud, 91100 Corbeil-Essonnes (FR); STEVERLYNCK, Céline, 94500 Champigny-sur-Marne (FR)
(74) Representative: Icosa

(57) **Abstract**

The present invention relates to a composition comprising a phosphorothioate oligonucleotide and at least one fatty acid and/or at least one emulsifying agent, wherein said composition is sterile and wherein said composition comprises an agent comprising a thiol group, preferably said composition is an ophthalmic composition; to a method for obtaining the same and to the therapeutic use thereof.

SEQUENCE LISTING

<110> GENE SIGNAL INTERNATIONAL SA
      Al-Mahmood , Salman

<120> STERILE EMULSION COMPRISING A STABLE PHOSPHOROTHIOATE OLIGONUCLEOTIDE

<130> CV - 539/EP

<160> 28

<170> BiSSAP 1.0

<210> 1
<211> 150
<212> DNA
<213> Artificial Sequence

<220>
<221> source
<222> 1..150
<223> /mol_type="DNA"
      /note="IRS-1 antisense"
      /organism="Artificial Sequence"

<400> 1
tagtactcga ggcgcgccgg gcccccagcc tcgctggccg cgcgcagtac gaagaagcgt      60

ttgtgcatgc tcttgggttt gcgcaggtag cccaccttgc gcacgtccga gaagccatcg     120

ctctccggag ggctcgccat gctgccaccg                                      150


<210> 2
<211> 25
<212> DNA
<213> Artificial Sequence

<220>
<221> source
<222> 1..25
<223> /mol_type="DNA"
      /note="IRS-1 antisense"
      /organism="Artificial Sequence"

<400> 2
tctccggagg gctcgccatg ctgct                                           25


<210> 3
<211> 25
<212> DNA
<213> Artificial Sequence

<220>
<221> source
<222> 1..25
<223> /mol_type="DNA"
      /note="IRS-1 antisense"
      /organism="Artificial Sequence"

<400> 3
tatccggagg gctcgccatg ctgct                                           25

<210> 4
<211> 24
<212> DNA
<213> Artificial Sequence

<220>
<221> source
<222> 1..24
<223> /mol_type="DNA"
      /note="IRS-1 antisense"
      /organism="Artificial Sequence"

<400> 4
tctccggagg gctcgccatg ctgc                                              24


<210> 5
<211> 23
<212> DNA
<213> Artificial Sequence

<220>
<221> source
<222> 1..23
<223> /mol_type="DNA"
      /note="IRS-1 antisense"
      /organism="Artificial Sequence"

<400> 5
tctccggagg gctcgccatg ctg                                               23


<210> 6
<211> 22
<212> DNA
<213> Artificial Sequence

<220>
<221> source
<222> 1..22
<223> /mol_type="DNA"
      /note="IRS-1 antisense"
      /organism="Artificial Sequence"

<400> 6
tctccggagg gctcgccatg ct                                                22


<210> 7
<211> 21
<212> DNA
<213> Artificial Sequence

<220>
<221> source
<222> 1..21
<223> /mol_type="DNA"
      /note="IRS-1 antisense"
      /organism="Artificial Sequence"

<400> 7

tctccggagg gctcgccatg c                                                21

<210> 8
<211> 20
<212> DNA
<213> Artificial Sequence

<220>
<221> source
<222> 1..20
<223> /mol_type="DNA"
      /note="IRS-1 antisense"
      /organism="Artificial Sequence"

<400> 8
tctccggagg gctcgccatg                                                  20

<210> 9
<211> 19
<212> DNA
<213> Artificial Sequence

<220>
<221> source
<222> 1..19
<223> /mol_type="DNA"
      /note="IRS-1 antisense"
      /organism="Artificial Sequence"

<400> 9
tctccggagg gctcgccat                                                   19

<210> 10
<211> 24
<212> DNA
<213> Artificial Sequence

<220>
<221> source
<222> 1..24
<223> /mol_type="DNA"
      /note="IRS-1 antisense"
      /organism="Artificial Sequence"

<400> 10
ctccggaggg ctcgccatgc tgct                                             24

<210> 11
<211> 23
<212> DNA
<213> Artificial Sequence

<220>
<221> source
<222> 1..23
<223> /mol_type="DNA"
      /note="IRS-1 antisense"
      /organism="Artificial Sequence"

<400> 11
tccggagggc tcgccatgct gct                                              23


<210> 12
<211> 22
<212> DNA
<213> Artificial Sequence

<220>
<221> source
<222> 1..22
<223> /mol_type="DNA"
      /note="IRS-1 antisense"
      /organism="Artificial Sequence"

<400> 12
ccggagggct cgccatgctg ct                                               22


<210> 13
<211> 21
<212> DNA
<213> Artificial Sequence

<220>
<221> source
<222> 1..21
<223> /mol_type="DNA"
      /note="IRS-1 antisense"
      /organism="Artificial Sequence"

<400> 13
cggagggctc gccatgctgc t                                                21


<210> 14
<211> 20
<212> DNA
<213> Artificial Sequence

<220>
<221> source
<222> 1..20
<223> /mol_type="DNA"
      /note="IRS-1 antisense"
      /organism="Artificial Sequence"

<400> 14
ggagggctcg ccatgctgct                                                  20


<210> 15
<211> 19
<212> DNA
<213> Artificial Sequence

<220>
<221> source
<222> 1..19
<223> /mol_type="DNA"
      /note="IRS-1 antisense"
      /organism="Artificial Sequence"

<400> 15
gagggctcgc catgctgct                                                    19


<210> 16
<211> 18
<212> DNA
<213> Artificial Sequence

<220>
<221> source
<222> 1..18
<223> /mol_type="DNA"
      /note="IRS-1 antisense"
      /organism="Artificial Sequence"

<400> 16
agggctcgcc atgctgct                                                     18


<210> 17
<211> 16
<212> DNA
<213> Artificial Sequence

<220>
<221> source
<222> 1..16
<223> /mol_type="DNA"
      /note="IRS-1 antisense"
      /organism="Artificial Sequence"

<400> 17
ggctcgccat gctgct                                                       16


<210> 18
<211> 15
<212> DNA
<213> Artificial Sequence

<220>
<221> source
<222> 1..15
<223> /mol_type="DNA"
      /note="IRS-1 antisense"
      /organism="Artificial Sequence"

<400> 18
gctcgccatg ctgct                                                        15


<210> 19
<211> 14
<212> DNA
<213> Artificial Sequence

<220>
<221> source
<222> 1..14
<223> /mol_type="DNA"
      /note="IRS-1 antisense"

/organism="Artificial Sequence"

<400> 19
ctcgccatgc tgct                                                          14


<210> 20
<211> 13
<212> DNA
<213> Artificial Sequence

<220>
<221> source
<222> 1..13
<223> /mol_type="DNA"
      /note="IRS-1 antisense"
      /organism="Artificial Sequence"

<400> 20
tcgccatgct gct                                                           13


<210> 21
<211> 12
<212> DNA
<213> Artificial Sequence

<220>
<221> source
<222> 1..12
<223> /mol_type="DNA"
      /note="IRS-1 antisense"
      /organism="Artificial Sequence"

<400> 21
cgccatgctg ct                                                            12


<210> 22
<211> 24
<212> DNA
<213> Artificial Sequence

<220>
<221> source
<222> 1..24
<223> /mol_type="DNA"
      /note="IRS-1 antisense"
      /organism="Artificial Sequence"

<400> 22
tatccggagg gctcgccatg ctgc                                               24


<210> 23
<211> 23
<212> DNA
<213> Artificial Sequence

<220>
<221> source
<222> 1..23
<223> /mol_type="DNA"

/note="IRS-1 antisense"
      /organism="Artificial Sequence"

<400> 23
tatccggagg gctcgccatg ctg                                              23


<210> 24
<211> 22
<212> DNA
<213> Artificial Sequence

<220>
<221> source
<222> 1..22
<223> /mol_type="DNA"
      /note="IRS-1 antisense"
      /organism="Artificial Sequence"

<400> 24
tatccggagg gctcgccatg ct                                               22


<210> 25
<211> 21
<212> DNA
<213> Artificial Sequence

<220>
<221> source
<222> 1..21
<223> /mol_type="DNA"
      /note="IRS-1 antisense"
      /organism="Artificial Sequence"

<400> 25
tatccggagg gctcgccatg c                                                21


<210> 26
<211> 20
<212> DNA
<213> Artificial Sequence

<220>
<221> source
<222> 1..20
<223> /mol_type="DNA"
      /note="IRS-1 antisense"
      /organism="Artificial Sequence"

<400> 26
tatccggagg gctcgccatg                                                  20


<210> 27
<211> 19
<212> DNA
<213> Artificial Sequence

<220>
<221> source
<222> 1..19

<223> /mol_type="DNA"
      /note="IRS-1 antisense"
      /organism="Artificial Sequence"

<400> 27
tatccggagg gctcgccat                                                    19


<210> 28
<211> 24
<212> DNA
<213> Artificial Sequence

<220>
<221> source
<222> 1..24
<223> /mol_type="DNA"
      /note="IRS-1 antisense"
      /organism="Artificial Sequence"

<400> 28
atccggaggg ctcgccatgc tgct                                              24

## Description

### FIELD OF INVENTION

The present invention relates to the therapeutic use of oligonucleotides and aims at solving the problem of the stability of said oligonucleotides in a composition, in particular in a composition comprising at least one fatty acid and/or at least one emulsifying agent. More specifically, the present invention relates to a sterile composition comprising at least one fatty acid and/or at least one emulsifying agent, a phosphorothioate oligonucleotide and an agent containing a thiol group.

### BACKGROUND OF INVENTION

Oligonucleotides are commonly used as laboratory tools and increasingly used as therapeutic agents. Oligonucleotides are characterized by their specificity, as they are able to recognize and bind to a specific target, for example through sequence complementarity by virtue of Watson-Crick base pairing. Antisense oligonucleotides, siRNAs and shRNAs, the most common oligonucleotides with therapeutic purposes, are thus able to modulate the expression of a target gene. In particular, antisense oligonucleotides bind to a specific mRNA target and induce its degradation through the recruitment of RNase H, a ubiquitous enzyme that hydrolyzes the RNA strand of RNA/DNA hybrids. Alternatively some antisense oligonucleotides may act as "steric blockers" as they block the access of cellular machinery to their RNA target. Antisense oligonucleotides are useful in the treatment of many disorders, including cancer, metabolic diseases, inflammatory diseases and angiogenesis related diseases.

Treatments consisting in the administration of an oligonucleotide to a human subject require compositions in which the oligonucleotide is stable. Unmodified oligonucleotides are susceptible to degradation by both intracellular and extracellular nucleases. Chemical modifications of the natural phosphodiester backbone were thus developed, and are currently commonly used to increase the stability of the modified oligonucleotides. In particular phosphorothioate oligonucleotides are known to be more resistant to degradation by nucleases. Phosphorothioate antisense oligonucleotides are able to activate RNase H activity and thus can induce the degradation of their target mRNA.

Depending on the disease treated, different ways of administration and correspondingly different types of composition are used. Phosphorothioate oligonucleotides are very stable in aqueous solutions. However, administration of oligonucleotides has also been envisaged in the form of emulsions, creams or any bi- or multiphasic other formulations, in particular for topical administration, in order to ensure a sufficient exposure of the target tissue to the active oligonucleotides. For example, a topical application of an emulsion may thus be required for the treatment of some diseases of the eyes. Being applied in an emulsion rather than in an aqueous solution may prevent the hydrophilic oligonucleotides from being readily absorbed in the vitreous humor.

However stability issues arise with phosphorothioate nucleotides in emulsions. Indeed phosphorothioate oligonucleotides are susceptible to desulfurization through the action of peroxide radicals generated from excipients present in the compositions. WO03005822 presents how the addition of antioxidants which partition into the aqueous phase of a bi- or multiphasic topical formulation prevents desulfurization of phosphorothioate internucleoside linkages.

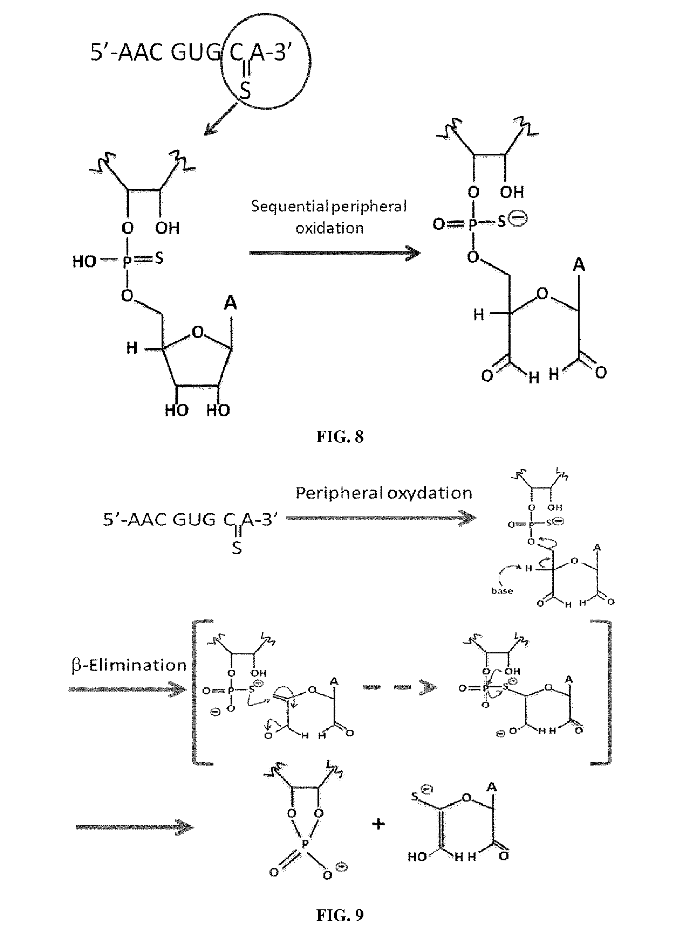

It should be noted that the bi- or multiphasic compositions comprising a phosphorothioate oligonucleotide and an antioxidant described in WO03005822 were not submitted to temperatures higher than 40°C. However some treatments, such as an ophthalmic application, require the administration of a sterile emulsion obtained by autoclaving, i.e. sterilization by saturated steam under pressure (more than 100°C). Upon autoclaving, the Applicant demonstrated that phosphorothioate oligonucleotides may be subjected to β-elimination following sequential peripheral oxidation, as shown in **Figures 8** and **9****.** Without willing to be bound to a theory, the Applicant suggests that exposition of fatty acids and/or emulsifying agents of the emulsion to high temperatures generate highly reactive chemical entities and/or free radicals which could lead to the degradation of the phosphorothioate oligonucleotide.

Thus there is still a need for a sterile composition comprising at least one fatty acid and/or at least one emulsifying agent where the phosphorothioate oligonucleotide is stable.

### SUMMARY

The present invention thus relates to a composition comprising a phosphorothioate oligonucleotide and at least one fatty acid and/or at least one emulsifying agent, wherein said composition is sterile and wherein said composition comprises an agent comprising a thiol group, preferably said composition is an ophthalmic composition.

In one embodiment, the composition is an emulsion, preferably an oil-in-water emulsion or a water-in-oil-in-water emulsion.

In one embodiment, the agent comprising a thiol group is selected from the group comprising lipoic acid, DL-cysteine, N-acetylcysteine, glutathione, 2-mercapto-5-benzimidazole salts and 2-mercaptoethanesulfonic acid salts, preferably the agent comprising a thiol group is lipoic acid, DL-cysteine or N-acetylcysteine.

In one embodiment, all phosphodiester linkages of the phosphorothioate oligonucleotide are replaced by phosphorothioate linkages.

In one embodiment, the phosphorothioate oligonucleotide is selected from the group comprising antisense oligonucleotides, siRNA, shRNA, ribozymes, aptamers, molecular decoys and RNA-DNA hybrid molecules, preferably said phosphorothioate oligonucleotide is an antisense oligonucleotide.

In one embodiment, the phosphorothioate oligonucleotide is an antisense oligonucleotide specific for IRS-1 (insulin receptor substrate-1), preferably the IRS-1 antisense phosphorothioate oligonucleotide is a sequence of at least 12 nucleotides, of SEQ ID NO: 1, more preferably the IRS-1 antisense phosphorothioate oligonucleotide is SEQ ID NO: 2 or a function-conservative derivative thereof.

In one embodiment, the function-conservative derivative of SEQ ID NO: 2 comprises from 9 to 50 nucleotides, has at least about 75% identity compared to SEQ ID NO: 2 and conserves the capacity of inhibiting IRS-1 expression as SEQ ID NO: 2, preferably said function-conservative derivative of SEQ ID NO: 2 is selected from SEQ ID NO: 3 to SEQ ID NO: 28.

In one embodiment, the phosphorothioate oligonucleotide is stable for at least 1 day at room temperature, and/or the phosphorothioate oligonucleotide is stable for at least 10 minutes at 60°C.

In one embodiment, said composition is an emulsion comprising
- an aqueous phase, wherein the amount of the aqueous phase in the emulsion ranges from about 70 to 99% in weight to the total weight of the emulsion; and
- an oily phase, wherein the amount of the oily phase in the emulsion ranges from about 1 to 30% in weight to the total weight of the emulsion;
wherein the aqueous phase comprises:
- a phosphorothioate oligonucleotide, preferably in an amount ranging from about 0.01% to about 3% in weight to the total weight of the emulsion; or in an amount ranging from about 0.01% to about 3% in weight to the total weight of the aqueous phase of the emulsion, wherein said phosphorothioate oligonucleotide is preferably an IRS-1 antisense;
- an agent containing a thiol group, preferably in an amount ranging from about 0.5% to about 5% in weight to the total weight of the composition; or in an amount ranging from about 0.5% to about 5% in weight to the total weight of the aqueous phase of the emulsion of the invention ;
- at least one polymer of acrylic acid, preferably in an amount ranging from about 0.01 % to about 0.1 % in weight to the total weight of the emulsion;
- NaOH in an amount sufficient for buffering the emulsion at a pH ranging from about 6 to about 7, such as, for example, ranging from about 0.1 to about 0.5% in weight to the total weight of the emulsion; and
wherein the oily phase comprises:
- Medium Chain Triglycerides (MCT), preferably in an amount ranging from about 1 to about 20% in weight to the total weight of the emulsion;
- a mixture of glyceryl stearate and of PEG-75, preferably in an amount ranging from about 1% to about 10% in weight to the total weight of the emulsion; and
- cetyl alcohol, preferably in an amount ranging from about 0.5 to about 25% in weight to the total weight of the emulsion.

The present invention further relates to a pharmaceutical composition comprising a composition as described hereinabove and at least one pharmaceutically acceptable excipient.

The present invention further relates to a medicament comprising a composition as described hereinabove.

The present invention further relates to a method for obtaining a sterile composition comprising a phosphorothioate oligonucleotide and at least one fatty acid and/or at least one emulsifying agent, wherein said method comprises:
- mixing the components of the composition and an agent comprising a thiol group;
- autoclaving the composition resulting from the previous step, and
- adding the phosphorothioate oligonucleotide to the autoclaved composition, preferably under sterile conditions.

In one embodiment, the autoclaving step comprises heating the emulsion at a temperature ranging from about 100°C to about 130°C for a period ranging from about 10 to about 30 minutes, preferably at about 121°C for about 20 minutes.

The present invention further relates to a composition obtained by the method as described hereinabove.

The present invention further relates to a composition as described hereinabove, wherein the phosphorothioate oligonucleotide is an IRS-1 antisense, for treating an angiogenic disorder.

### DEFINITIONS

In the present invention, the following terms have the following meanings:
- **"Ophthalmic composition":** refers to sterile liquid, semi-solid or solid preparations intended for administration upon the eyeball and/or to the conjunctiva, or for insertion in the conjunctival sac or for administration into the posterior segment of the eye. As used herein, the term "posterior segment of the eye" refers to the back two third of the eye, comprising the anterior hyaloids membrane and the structures behind it (vitreous humor, retina, choroid, optic nerve). In particular, an ophthalmic composition may be administered into the vitreous humor, for example by intravitreous injection. Examples of ophthalmic compositions include, but are not limited to, eye drops, eye lotions, powders for eye drops and powders for eye lotions, and compositions to be injected into the conjunctival sac or into the vitreous humor.
- **"Eye drops"** refers to sterile aqueous or oily solutions, emulsions or suspensions of one or more active substances intended for instillation into the eye. According to the present invention, the one or more active substances comprises at least one phosphorothioate oligonucleotide.
- **"Eye lotions"** refers to sterile aqueous or oily solutions intended for use in rinsing or bathing the eye or for impregnating eye dressings.
- **"Oligonucleotide":** refers to an acid nucleic molecule, i.e. a polymer of ribonucleic acids or deoxyribonucleic acids, either single- or double-stranded. In one embodiment, the length of an oligonucleotide ranges from about 5 to about 200 nucleotides, preferably from about 7 to 100 nucleotides, more preferably from 10 to 70 nucleotides and even more preferably from 12 to 30 nucleotides.
- **"Phosphorothioate oligonucleotide"** refers to an oligonucleotide in which at least one non-bridging oxygen on the phosphate backbone of the nucleotides has been replaced by a sulfur atom to form phosphorothioate internucleotide linkages (instead of the natural phosphodiester internucleotide linkages). Such chemical modification protects oligonucleotides from degradation by nucleases, both intracellular and extracellular. Phosphorothiotate linkages have also been reported to increase the cellular uptake of the oligonucleotides. They can also bind to serum proteins slowing excretion by the kidneys.
- **"Fatty acid":** refers to a carboxylic acid with a long aliphatic tail (chain), such as, for example, from 4 to 36 atoms of carbon, which is either saturated or unsaturated.
- **"Thiol":** refers to a group -SH.
- **"Pharmaceutically acceptable excipient":** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.
- **"About":** preceding a figure means plus or minus 10% of the value of said figure.
- **"Therapeutically effective amount":** means level or amount of agent that is aimed at, without causing significant negative or adverse side effects to the target, (1) delaying or preventing the targeted disease, disorder, or condition; (2) slowing down or stopping the progression, aggravation, or deterioration of one or more symptoms of the targeted disease, disorder, or condition; (3) bringing about ameliorations of the symptoms of the targeted disease, disorder, or condition; (4) reducing the severity or incidence of the targeted disease, disorder, or condition; or (5) curing the targeted disease, disorder, or condition. A therapeutically effective amount may be administered prior to the onset of the targeted disease, disorder, or condition, for a prophylactic or preventive action. Alternatively or additionally, the therapeutically effective amount may be administered after initiation of the targeted disease, disorder, or condition, for a therapeutic action.
- **"Treating"** or **"treatment"** refers to both therapeutic treatment and prophylactic or preventative measures; wherein the object is to prevent or slow down (lessen) the targeted disease, disorder, or condition. Those in need of treatment include those already with the disorder as well as those prone to have the disorder or those in whom the disorder is to be prevented. A subject or mammal is successfully "treated" for an infection if, after receiving a therapeutic amount of a composition according to the present invention, the subject shows observable and/or measurable reduction in or absence of one or more of the following: reduction in the number of pathogenic cells; reduction in the percent of total cells that are pathogenic; and/or relief to some extent, of one or more of the symptoms associated with the specific disease or condition; reduced morbidity and mortality, and improvement in quality of life issues. The above parameters for assessing successful treatment and improvement in the disease are readily measurable by routine procedures familiar to a physician.
- **"Subject"** refers to a mammal, preferably a human. In one embodiment, a subject may be a "patient", i.e. a warm-blooded animal, more preferably a human, who/which is awaiting the receipt of, or is receiving medical care or was/is/will be the object of a medical procedure, or is monitored for the development of a disease.

### DETAILED DESCRIPTION

One object of the invention is a composition comprising a phosphorothioate oligonucleotide and at least one fatty acid and/or at least one emulsifying agent, wherein said composition is sterile and wherein said composition comprises an agent comprising a thiol group.

In one embodiment, the composition of the invention is an ophthalmic composition.

According to the invention, the composition of the invention provides stability to the phosphorothioate oligonucleotide. Without willing to be bound to any theory, the Applicant suggests that the presence of an agent comprising a thiol group prevents the degradation of the phosphorothioate oligonucleotide. In particular, the presence of an agent comprising a thiol group may prevent the oligonucleotide from attack by chemical bases liberated from fatty acids and/or from emulsifying agents during an autoclaving step.

Examples of compositions comprising at least one fatty acid and/or at least one emulsifying agent include, but are not limited to, gels, ointments, micelles and emulsions.

In one embodiment of the invention, the composition comprising at least one fatty acid and/or at least one emulsifying agent is an emulsion such as a water-in-oil emulsion, an oil-in-water emulsion, a water-in-oil-in-water emulsion or any multiphasic emulsion. Preferably, the composition comprising at least one fatty acid and/or at least one emulsifying agent is a water-in-oil-in-water emulsion.

In one embodiment, the emulsion is cationic. In another embodiment, the emulsion is anionic.

In one embodiment, the emulsion of the invention comprises an aqueous phase and an oil phase dispersed in the aqueous phase, wherein:
- the percentage of the aqueous phase ranges from about 70 to about 99% in weight to the total weight of the emulsion, preferably from about 75% to about 85% w/w, more preferably is of about 81.5%; and wherein
- the percentage of the oil phase ranges from about 1 to about 30% in weight to the total weight of the emulsion, preferably from about 10 to about 20% w/w, more preferably is of about 18.5%.

In one embodiment, the oil-in-water emulsion described hereinabove further comprises an aqueous phase inside the oil droplets dispersed in the aqueous phase, and is therefore a water-in-oil-in-water emulsion.

In one embodiment of the invention, the phosphorothioate oligonucleotide is present in the aqueous phase of the emulsion. In one embodiment, the emulsion is a water-in-oil-in-water emulsion, and the phosphorothioate oligonucleotide is present both in the aqueous phase surrounding the oil droplets and in the aqueous phase inside the oil droplets.

In one embodiment of the invention, the agent comprising a thiol group is present in the aqueous phase of the emulsion. In one embodiment, the emulsion is a water-in-oil-in-water emulsion, and the agent comprising a thiol group is present both in the aqueous phase surrounding the oil droplets and in the aqueous phase inside the oil droplets.

Examples of fatty acids include, but are not limited to: (1) saturated fatty acids, which have no C=C moieties and include myristic acid, palmitic acid, stearic acid, arachidic acid, behenic acid, lignoceric acid; and (2) unsaturated fatty acids, including the following: monounsaturated fatty acids, which have one C=C group such as palmitoleic acid, oleic acid, and nervonic acid; diunsaturated fatty acids, which have two C=C groups, such as linoleic acid; triunsaturated fatty acids, which have three C=C groups, such as [alpha]-linolenic acid and [gamma]-linolenic acid; tetraunsaturated fatty acids, which have four C=C groups, such as arachidonic acid; and pentaunsaturated fatty acids, which have five C=C groups, such as eicosapentaenoic acid. The following may also be used: Lauric Acid; 14 carbon fatty acids such as myristic acid; 16 carbon fatty acids such as palmitic and palmitoleic acid; 18 carbon fatty acids such as stearic acid, oleic acid, linoleic acid, [alpha]-linolenic acid, and [gamma]-linolenic acid; 20 carbon fatty acids such as eicosapentaenoic acid; 22 carbon fatty acids such as arachidic acid; and 24 carbon fatty acids such as lignoceric acid and nervonic acid.

In one embodiment, the composition of the invention is an emulsion, and the at least one fatty acid is comprised in the oil phase of the emulsion. Examples of oil that may be used are listed below.

Examples of agents comprising a thiol group include, but are not limited to, DL-cysteine, N-acetylcysteine, lipoic acid, glutathione, 2-mercapto-5-benzimidazole salts, 2-mercaptoethanesulfonic acid salts. Preferably, the agent comprising a thiol group is selected from the group comprising DL-cysteine, N-acetylcysteine and lipoic acid.

Preferably, the composition of the invention is an emulsion, and the agent comprising a thiol group is comprised in the aqueous phase of the emulsion.

In one embodiment, the amount of agent comprising a thiol group in the composition of the invention ranges from about 0.5% to about 5% in weight to the total weight of the composition, preferably from about 1 to about 3% w/w, more preferably is of about 2% w/w.

In one embodiment, the amount of agent comprising a thiol group in the emulsion of the invention ranges from about 0.5% to about 5% in weight to the total weight of the aqueous phase, preferably from about 1 to about 3% w/w, more preferably is of about 2.45% w/w.

In one embodiment, the phosphorothioate oligonucleotide is an active oligonucleotide that may be used for therapeutic purposes.

In one embodiment, at least one (such as, for example, 1, 2, 3, 4, 5, 6, 7, 8, 9 or 10) phosphodiester linkage of the oligonucleotide of the invention is replaced by a phosphorothioate linkage. In another embodiment, the first phosphodiester linkage in 5' and the first phosphodiester linkage in 3' of the oligonucleotide of the invention are replaced by a phosphorothioate linkage. Preferably, however, all phosphodiester linkages of the oligonucleotide of the invention are replaced by phosphorothioate linkages.

Examples of phosphorothioate oligonucleotides include, but are not limited to, antisense oligonucleotides, siRNAs, shRNAs, ribozymes, aptamers, molecular decoys and RNA-DNA hybrid molecules.

Antisense oligonucleotides are commonly used to modulate gene expression through their hybridization to a specific RNA target by virtue of Watson-Crick base pairing. In particular, antisense oligonucleotides bind to a specific mRNA target and may induce its degradation through the recruitment of RNase H, a ubiquitous enzyme that hydrolyzes the RNA strand of RNA/DNA hybrids. Alternatively some antisense oligonucleotides act as "steric blockers" as they block the access of cellular machinery to their RNA target.

Both siRNAs and shRNAs are able to modulate gene expression through the gene silencing mechanism known as RNA interference in which a small RNA duplex associates with the RNA-induced silencing complex (RISC) to lead the RISC to a specific target mRNA. siRNAs are small double-stranded RNA molecules directly delivered to the cells while shRNAs are artificial RNA molecules with a tight hairpin turn usually delivered to the cells via a plasmid or a vector and further processed within the cells.

Ribozymes are RNA molecules capable of catalyzing specific biochemical reactions. Artificial ribozymes have been designed to target the RNA of specific viruses.

Aptamers are short synthetic single-stranded oligonucleotides that specifically bind to various molecular targets such as small molecules, proteins, nucleic acids, and even cells and tissues via a mechanism other than Watson-Crick base-pairing. They are essentially a chemical equivalent of antibodies.

Molecular decoys are short double-stranded synthetic polynucleotides with high affinity for a regulatory protein, such as a transcription factor, that can be used to compete with the natural nucleic acid and attenuate the effects of the regulatory protein.

RNA-DNA hybrid molecules, also known as chimeric oligonucleotides (chimeras), have been shown to alter or repair single bases in plant and animal genomes.

In one embodiment of the invention, the bioactive phosphorothioate oligonucleotide is a phosphorothioate antisense oligonucleotide.

In one embodiment of the invention, the phosphorothioate antisense oligonucleotide inhibits the expression of IRS-1 (insulin receptor substrate-1).

In one embodiment of the invention, the IRS-1 antisense oligonucleotide is a sequence of at least 12 nucleotides, preferably at least 12 contiguous oligonucleotides of SEQ ID NO: 1: 5'-TAGTACTCGAGGCGCGCCGGGCCCCCAGCCTCGCTGGCCGCGCGCAGTACG AAGAAGCGTTTGTGCATGCTCTTGGGTTTGCGCAGGTAGCCCACCTTGCGCA CGTCCGAGAAGCCATCGCTCTCCGGAGGGCTCGCCATGCTGCCACCG-3'.

In one embodiment of the invention, the IRS-1 antisense oligonucleotide is a sequence comprising or consisting of at least 12 contiguous nucleotides of SEQ ID NO: 1, preferably at least 13, 14, 15, 16, 17, 18, 19, 20, 21, 22, 23, 24, 25, 26, 27, 28, 29, 30 contiguous nucleotides of SEQ ID NO: 1.

In one embodiment of the invention, the IRS-1 antisense oligonucleotide is GS-101. According to the invention, GS-101 is an antisense oligonucleotide having the sequence SEQ ID NO: 2 (5'-TCTCCGGAGGGCTCGCCATGCTGCT-3').

In one embodiment, the IRS-1 antisense oligonucleotide is a function conservative sequence of SEQ ID NO: 2, wherein said function conservative sequence comprises from 9 to 50, 12 to 45, 15 to 40, 20 to 35, or 25 to 30 nucleotides that has 75%, 80%, 85%, 90%, 95% or more than 95%, 96%, 97%, 98%, 99% of identity compared to SEQ ID NO: 2 and that conserves the capacity of inhibiting IRS-1 expression as SEQ ID NO: 2.

In one embodiment, the function conservative sequence of SEQ ID NO: 2 comprises 9, 10, 11, 12, 13, 14, 15, 16, 17, 18, 19, 20, 21, 22, 23, 24, 25, 26, 27, 28, 29 or 30 nucleotides.

The term "identity" or "identical", when used in a relationship between the sequences of two or more nucleotidic sequences, refers to the degree of sequence relatedness between nucleotidic sequences, as determined by the number of matches between strings of two or more bases. "Identity" measures the percent of identical matches between the smaller of two or more sequences with gap alignments (if any) addressed by a particular mathematical model or computer program (i.e., "algorithms"). Identity of related nucleotidic sequences can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, and FASTA (Altschul et al., J. MoI. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

An example of a function conservative sequence of SEQ ID NO: 2 is SEQ ID NO: 3 (5'-TATCCGGAGGGCTCGCCATGCTGCT-3').

Other examples of function conservative sequences of SEQ ID NO: 2 include, but are not limited to, the following sequences:
5'-TCTCCGGAGGGCTCGCCATGCTGC-3' (SEQ ID NO: 4)
5'-TCTCCGGAGGGCTCGCCATGCTG-3' (SEQ ID NO: 5)
5'-TCTCCGGAGGGCTCGCCATGCT-3' (SEQ ID NO: 6)
5'-TCTCCGGAGGGCTCGCCATGC-3' (SEQ ID NO: 7)
5'-TCTCCGGAGGGCTCGCCATG-3' (SEQ ID NO: 8)
5'-TCTCCGGAGGGCTCGCCAT-3' (SEQ ID NO: 9)
5'-CTCCGGAGGGCTCGCCATGCTGCT-3' (SEQ ID NO: 10)
5'-TCCGGAGGGCTCGCCATGCTGCT-3' (SEQ ID NO: 11)
5'-CCGGAGGGCTCGCCATGCTGCT-3' (SEQ ID NO: 12)
5'-CGGAGGGCTCGCCATGCTGCT-3' (SEQ ID NO: 13)
5'-GGAGGGCTCGCCATGCTGCT-3' (SEQ ID NO: 14)
5'-GAGGGCTCGCCATGCTGCT-3' (SEQ ID NO: 15)
5'-AGGGCTCGCCATGCTGCT-3' (SEQ ID NO: 16)
5'-GGCTCGCCATGCTGCT-3' (SEQ ID NO: 17)
5'-GCTCGCCATGCTGCT-3' (SEQ ID NO: 18)
5'-CTCGCCATGCTGCT-3' (SEQ ID NO: 19)
5'-TCGCCATGCTGCT-3' (SEQ ID NO: 20)
5'-CGCCATGCTGCT-3' (SEQ ID NO: 21).
5'-TATCCGGAGGGCTCGCCATGCTGC-3' (SEQ ID NO: 22)
5'-TATCCGGAGGGCTCGCCATGCTG-3' (SEQ ID NO: 23)
5'-TATCCGGAGGGCTCGCCATGCT-3' (SEQ ID NO: 24)
5'-TATCCGGAGGGCTCGCCATGC-3' (SEQ ID NO: 25)
5'-TATCCGGAGGGCTCGCCATG-3' (SEQ ID NO: 26)
5'-TATCCGGAGGGCTCGCCAT-3' (SEQ ID NO: 27)
5'-ATCCGGAGGGCTCGCCATGCTGCT-3' (SEQ ID NO: 28)

In one embodiment of the invention, said function conservative sequence of SEQ ID NO: 2 of 25, 30, 35, 40, 45 or 50 nucleotides may be a sequence comprising SEQ ID NO: 2 or SEQ ID NO: 3 between other nucleic acids in C-terminal and N-terminal. Said function conservative sequence may also be a 9 to 12 contiguous nucleotides fragment of SEQ ID NO: 2 or SEQ ID NO: 3.

The phosphorothioate oligonucleotide of the invention, such as, for example, the inhibitors of IRS-1 as hereinabove described, may be synthesized by all methods well known by the person skilled in the art, such as chemical synthesis.

In one embodiment, the phosphorothioate oligonucleotide of the invention may be sterilized, such as, for example, by filtration, preferably using a filter with a size ranging from about 0.2 to about 0.8 µm, preferably from about 0.4 to about 0.5 µm, and more preferably through a 0.45 µm filter.

In one embodiment, the composition of the invention comprises an amount of at least one phosphorothioate oligonucleotide ranging from about 0.01% to about 3% in weight to the total weight of the composition, preferably from about 0.04% to about 2% w/w and more preferably is of about 1.72% w/w.

In another embodiment, the composition of the invention is an emulsion and comprises an amount of at least one phosphorothioate oligonucleotide ranging from about 0.01% to about 3% in weight to the total weight of the aqueous phase of the emulsion, preferably from about 0.04% to about 2.5% w/w and more preferably is of about 2.11% w/w.

In one embodiment, the composition of the invention comprises from about 0.40 mg/ml to about 1.75, preferably from about 0.50 mg/ml to about 1.75 mg/ml, more preferably from about 0.60 mg/ml to about 1.5 mg/ml of a phosphorothioate oligonucleotide, preferably of an IRS-1 antisense as hereinabove described.

In another embodiment, the composition of the invention comprises from about 0.70 mg/ml to about 1.25 mg/ml of a phosphorothioate oligonucleotide, preferably of an IRS-1 antisense as hereinabove described.

In another embodiment, the composition of the invention comprises from about 0.80 mg/ml to about 1 mg/ml, preferably from about 0.8 to about 0.90 mg/ml of a phosphorothioate oligonucleotide, preferably of an IRS-1 antisense as hereinabove described.

In one embodiment, the composition of the invention comprises from about 0.40 mg/ml to about 0.50 mg/ml of a phosphorothioate oligonucleotide, preferably of an IRS-1 antisense as hereinabove described, preferably about 0.43 mg/ml.

In another embodiment, the composition of the invention comprises from about 0.80 mg/ml to about 0.90 mg/ml of a phosphorothioate oligonucleotide, preferably of an IRS-1 antisense as hereinabove described, preferably about 0.86 mg/ml.

In another embodiment, the composition of the invention comprises from about 1.60 mg/ml to about 1.80 mg/ml of a phosphorothioate oligonucleotide, preferably of an IRS-1 antisense as hereinabove described, preferably about 1.70 mg/ml.

In one embodiment, the composition of the invention comprises at least one oil which may be a vegetable oil, for example, castor oil, olive oil, soy oil, sesame oil, cotton seed oil, sweet almond oil or arachis oil; triglycerides, such as, for example, semi-synthetic oils (medium chains triglycerides (MCT) or long chain triglycerides); monoglycerides; diglycerides; oily fatty acids; isopropyl myristate; oily fatty alcohols; esters of sorbitol and fatty acids, oily sucrose esters, or a mineral oil, for example, liquid paraffin or petrolatum; and in general any oily substance which is physiologically tolerated and mixtures thereof.

In one embodiment, the composition of the invention is an emulsion, and the oil phase of the emulsion comprises MCT, i.e. a triglyceride oil in which the carbohydrate chain has about 8-12 carbon atoms. Example of MCT oil which may be used in emulsions of the present invention is Miglyol 812™ (supplied for example by Dynamit Novel, Sweden).

In one embodiment of the invention, the amount of the oil in the composition, preferably in the emulsion, ranges from about 1 to about 20% in weight to the total weight of the composition, preferably from about 5% to about 10% w/w, more preferably is of about 8% w/w.

In one embodiment of the invention, the amount of the oil in the emulsion ranges from about 25 to about 75% in weight to the total weight of the oily phase of the emulsion, preferably from about 35% to about 50% w/w, more preferably is of about 43.2% w/w.

In one embodiment of the invention, the composition comprises at least one emulsifying agent. Preferably, when the composition of the invention comprises at least one emulsifying agent, the composition is an emulsion. As used herein, the term "emulsifying agent" may be used interchangeably with surfactant.

In one embodiment, the emulsifying agent is in the aqueous and/or in the oil phase.

A surfactant may be used for assisting in dissolving an excipient or an active agent, dispersing a solid or liquid in a composition, enhancing wetting, modifying drop size, or a number of other purposes. Useful surfactants include, but are not limited to surfactants of the following classes: alcohols; amine oxides; block polymers; carboxylated alcohol or alkylphenol ethoxylates; carboxylic acids/fatty acids; ethoxylated alcohols; ethoxylated alkylphenols; ethoxylated aryl phenols; ethoxylated fatty acids; ethoxylated; fatty esters or oils (animal & veg.); fatty esters; fatty acid methyl ester ethoxylates; glycerol esters; glycol esters; lanolin-based derivatives; lecithin and lecithin derivatives; lignin and lignin derivatives; methyl esters; monoglycerides and derivatives; polyethylene glycols; polymeric surfactants; propoxylated & ethoxylated fatty acids, alcohols, or alkyl phenols; protein-based surfactants; sarcosine derivatives; sorbitan derivatives; sucrose and glucose esters and derivatives.

In particular, ethoxylate surfactants are useful. An ethoxylate surfactants is one that comprises the moiety -O(CH₂CH2O)ₙ-OH, wherein n is at least about 1.

In one embodiment n is from about 1 to about 10,000. In another embodiment, n is from 1 to about 1000. In another embodiment, n is from about 1 to about 500. Some ethoxylates contain one ethoxylate moiety. In other words, there is a single ethoxylate chain on each molecule.

Examples of surfactants with one ethoxylate moiety, include, but are not limited to, Ethoxylated alcohols wherein the alcohol has a single hydroxyl unit; alkylphenol ethoxylates; ethoxylated fatty acids; fatty acid methyl ester ethoxylates; polyethylene glycols; and the like.

Ethoxylates may comprise more than one ethoxylate moiety. In other words, there may be ethoxylate moieties attached to several different parts of the molecule. Examples include, but are not limited to: block polymers; ethoxylated oils; sorbitan derivatives; sucrose and glucose ethoxylates; and the like.

Block Polymers are polymers with the structure A-B-A', wherein A and A' are polyethylene chains of 1 or more ethylene units, and B is a polypropylene chain of one or more propylene units. Generally, but not necessarily, A and A' are approximately the same length.

In one embodiment, A and A' contain from about 2 to about 200 ethylene units. In another embodiment, A and A' contain from about 5 to about 100 ethylene units. In another embodiment, A and A' contain about 7 to about 15 ethylene units. In another embodiment, A and A' contain about 7, about 8, or about 12 ethylene units. In another embodiment, B contains from about 25 to about 100 propylene units. In another embodiment, B contains from about 30 to about 55 propylene units. In another embodiment, B contains about 30, about 34, or about 54 propylene units. In another embodiment, the molecular weight is from about 1000 to about 20000. In another embodiment, the molecular weight is from about 2000 to about 10000. In another embodiment, the molecular weight is about 2500, about 3000, about 3800, or about 8400.

Examples of block Polymers include, but are not limited to:
- Poloxalene: wherein A has about 12 ethylene oxide units, B has about 34 propylene oxide units, A' has about 12 ethylene oxide units, and the average molecular weight is about 3000.
- Poloxamer 182: wherein A has about 8 ethylene oxide units, B has about 30 propylene oxide units, A' has about 8 ethylene oxide units, and the average molecular weight is about 2500.
- Poloxamer 188: wherein A has about 75 ethylene oxide units, B has about 30 propylene oxide units, A' has about 75 ethylene oxide units, and the average molecular weight is about 8400.
- Poloxamer 331: wherein A has about 7 ethylene oxide units, B has about 54 propylene oxide units, A' has about 7 ethylene oxide units, and the average molecular weight is about 3800.

Examples of ethoxylated Alcohols include, but are not limited to:
- Ethoxylates of linear alcohols having from about 6 to about 20 carbon atoms. In one embodiment, the linear alcohol has from about 10 to about 16 carbon atoms. In another embodiment, n is from about 1 to about 100. In another embodiment, n is from about 1 to about 50. In another embodiment, n is from about 5 to about 50 ethylene oxide units. In another embodiment, n is from about 1 to about 20 ethylene oxide units. In another embodiment, n is from about 30 to about 50 ethylene oxide units.
- Ethoxylated Alkylphenols which are alkylphenols that are ethoxylated, i.e. the phenolic OH is replaced with an ethoxylate moiety. These include but are not limited to: octylphenol ethoxylate, i.e. C₈H₁₇Ph(OCH₂CH2O)ₙH. nonylphenol ethoxylate, i.e. C₉H₁₉Ph(OCH₂CH₂O)ₙH. alkyphenols of the above formula wherein n is from about 1 to about 100.
- Ethoxylated Fatty Acids, which include but are not limited to: ethoxylates which are esterified to form either: monoesters, i.e. RCO₂ (CH₂CH₂O)ₙOH, where RCO₂H is a fatty acid; or diesters , i.e. RCO₂(CH₂CH₂O)ₙC(=0)R.

Examples of fatty acids include, but are not limited to: (1) saturated fatty acids, which have no C=C moieties and include myristic acid, palmitic acid, stearic acid, arachidic acid, behenic acid, lignoceric acid; and (2) unsaturated fatty acids, including the following: monounsaturated fatty acids, which have one C=C group such as palmitoleic acid, oleic acid, and nervonic acid; diunsaturated fatty acids, which have two C=C groups, such as linoleic acid; triunsaturated fatty acids, which have three C=C groups, such as [alpha]-linolenic acid and [gamma]-linolenic acid; tetraunsaturated fatty acids, which have four C=C groups, such as arachidonic acid; and pentaunsaturated fatty acids, which have five C=C groups, such as eicosapentaenoic acid. The following may also be used: Lauric Acid; 14 carbon fatty acids such as myristic acid; 16 carbon fatty acids such as palmitic and palmitoleic acid; 18 carbon fatty acids such as stearic acid, oleic acid, linoleic acid, [alpha]-linolenic acid, and [gamma]-linolenic acid; 20 carbon fatty acids such as eicosapentaenoic acid; 22 carbon fatty acids such as arachidic acid; and 24 carbon fatty acids such as lignoceric acid and nervonic acid.

Ethoxylated Fatty Esters or Oils (Animal & Veg.) are products which result from reacting ethylene oxide with a fatty ester or an oil. When a fatty oil is used, the products is a mixture of ethoxylates of the fatty acids present in the oil, ethoxylates of glycerine, ethoxylates of mono and diglycerides, and the like. Specific examples include, but are not limited to: Ethoxylates of the following oils: Anise oil, Castor oil, Clove oil, Cassia oil, Cinnamon oil; Almond oil, Corn oil, Arachis oil, Cottonseed oil, Safflower oil, Maize oil, Linseed oil, Rapeseed oil, Soybean oil, Olive oil, Caraway oil, Rosemary oil, Peanut oil, Peppermint oil, Sunflower oil, Eucalyptus oil and Sesame oil; Coriander oil, Lavender oil, Citronella oil, Juniper oil, Lemon oil, Orange oil, Clary sage oil, Nutmeg oil, Tea tree oil, coconut oil, tallow oil, and lard. In one embodiment, from 1 to about 50 moles of ethylene oxide is used per mole of the oil triglyceride. In another embodiment, from about 30 to about 40 moles of ethylene oxide is used per mole of the oil triglyceride.

Ethylene oxide may also react with a fatty acid ester with a formula RCO₂R' to form RCO₂(CH₂CH₂O)ₙR'. Thus, surfactants having the formula RCO₂(CH₂CH₂O)ₙR', where RCO₂H is a fatty acid and R' is alkyl having from 1 to 6 carbons are contemplated. One embodiment is a fatty acid methyl ester ethoxylate, wherein R' is methyl.

In another embodiment, RCO₂H is Lauric Acid; a 14 carbon fatty acid such as myristic acid; a 16 carbon fatty acid such as palmitic and palmitoleic acid; an 18 carbon fatty acids such as stearic acid, oleic acid, linoleic acid, [alpha]-linolenic acid, and [gamma]-linolenic acid; a 20 carbon fatty acids such as eicosapentaenoic acid; a 22 carbon fatty acids such as arachidic acid; or a 24 carbon fatty acids such as lignoceric acid and nervonic acid. Polyethylene Glycols are ethoxylates that are unsubstituted, or terminated with oxygen on both ends, i.e. HO(CH₂CH₂O)ₙH,

Sorbitan Derivatives are ethoxylated sorbates having a fatty acid capping one or more of the ethoxylated chains. These include but are not limited to: (A) sorbitan derivatives wherein the total number of ethylene oxide units is from 3 to 30; (B) sorbitan derivatives wherein the total number of ethylene oxide units is 4, 5, or 20; (C) sorbitan derivatives wherein the capping acid is laurate, palmitate, stearate, or oleate; The sorbitan derivative may be a POE sorbitan monolaurate; a POE sorbitan dilaurate; a POE sorbitan trilaurate; a POE sorbitan monopalmitate; a POE sorbitan dipalmitate; a POE sorbitan tripalmitate; a POE sorbitan monostearate; a POE sorbitan distearate; a POE sorbitan tristearate; a POE sorbitan monooleate; a POE sorbitan dioleate; or a POE sorbitan trioleate; Specific examples include: POE (20) sorbitan monolaurate; POE (4) sorbitan monolaurate; POE (20) sorbitan monopalmitate; POE (20) monostearate; POE (20) sorbitan monostearate; POE (4) sorbitan monostearate; POE (20) sorbitan tristearate; POE (20) sorbitan monoleate; POE (20) sorbitan 15 monoleate; POE (5) sorbitan 10 monoleate; POE (20) sorbitan trioleate.

Sucrose and Glucose Esters and Derivatives include, but are not limited to, polyvinyl alcohol, povidone, hydroxypropyl methyl cellulose, poloxamers, carboxymethyl cellulose, hydroxyethyl cellulose, and acrylates (e.g. Pemulen(R)).

Other examples of suitable emulsifying agents include, but are not limited to naturally-occurring gums, for example, gum acacia or gum tragacanth; naturally-occurring phosphatides, for example, soya bean lecithin, and esters or partial esters derived from fatty acids and hexitol anhydrides, for example, sorbitan mono-oleate, and condensation products of the said partial esters with ethylene oxide, for example, polyoxyethylene sorbitan mono-oleate; sorbitan ester such as, for example, sorbitan stearate, sorbitan mono laurate, polyoxyethylene sorbitan mono oleate and sorbitan monopalmitate; bentonite; glycerin monostearate; glyceryl monooleate and propylene glycol monolaurate or mixtures thereof; glyceryl stearate; poloxamer 188; poloxamer 282; poloxamer 407; tyloxapol; vitamin E D-polyethylene glycol succinate; polyethylene glycol (PEG)(such as, for example, PEG 75); cetostearyl alcohol; cholesterol; ethylene glycol palmitostearate; lauric acid; myristic acid; myristyl alcohol; linoleic acid; oleic acid; palmitic acid; polysorbate 20 (Tween 20), sorbitan trioleate (Span 85), phospholipids such as egg lecithin stearic acid oleyl alcohol; and mixture thereof.

In one embodiment where the composition of the invention is an emulsion, the emulsifying agent is comprised in the oil phase. In one embodiment, the emulsion of the invention comprises glyceryl stearate and PEG75 as emulsifying agent. A mixture of glyceryl stearate and PEG75 is commercially available as Gelot 64®.

In one embodiment, the amount of emulsifying agent in the emulsion ranges from about 1% to about 10% in weight to the total weight of the emulsion, preferably from about 2.5 to about 5% w/w, more preferably is of about 3.5% w/w.

In one embodiment, the amount of emulsifying agent in the emulsion ranges from about 5% to about 40% in weight to the total weight of the oil phase of the emulsion, preferably from about 10 to about 30% w/w, more preferably is of about 18.9% w/w.

In another embodiment, the composition of the invention may further comprises another emulsifying agent such as a viscosity modifying agent. Examples of viscosity modifying agents include, but are not limited to, a hydrogel of sodium hyaluronate, polymers of acrylic acid, for example polymers of acrylic acids cross-linked with polyalkenyl ethers or divinyl glycol, such as, for example, Carbopol® gels, hydroxyethyl cellulose, dextran, carboxymethyl cellulose, polyethylene glycol, polyvinyl alcohol and collagen. In one embodiment, the composition of the invention is an emulsion, and the viscosity modifying agent is comprised in the aqueous phase. In an embodiment of the invention, the viscosity modifying agent is a Carbopol®, such as, for example, Carbopol® 980 NF. In said embodiment, the viscosity modifying agent is in the aqueous phase of the emulsion.

In one embodiment of the invention, the amount of the viscosity modifying agent in the composition, preferably in the emulsion ranges from about 0.01% to about 0.1% in weight to the total weight of the composition, preferably from about 0.03 to about 0.08% w/w, more preferably is of about 0.05% w/w.

In one embodiment of the invention, the amount of the viscosity modifying agent in the emulsion ranges from about 0.01% to about 0.2% in weight to the total weight of the aqueous phase of the emulsion, preferably from about 0.03 to about 0.1% w/w, more preferably is of about 0.061% w/w.

In one embodiment, the composition further comprises a thickening agent. Examples of suitable thickening agents include, but are not limited to, beeswax, hard paraffin and cetyl alcohol. Advantageously, said thickening agent is cetyl alcohol. In one embodiment the composition of the invention is an emulsion, and the thickening agent is comprised in the oil phase of the emulsion.

In one embodiment, the amount of thickening agent in the composition, preferably in the emulsion, ranges from about 0.1% to about 10% in weight to the total weight of the composition, preferably from about 0.5 to about 5% w/w, more preferably is of about 2% w/w.

In one embodiment, the amount of thickening agent in the emulsion ranges from about 1% to about 30% in weight to the total weight of the oily phase of the emulsion, preferably from about 5 to about 20% w/w, more preferably is of about 10.8% w/w.

In one embodiment, the composition further comprises an osmolality modifying agent. Examples of suitable osmolality modifying agents include, but are not limited to, NaCl, KCl, CaCl₂, glycerol, mannitol, alpha-trehalose and propylene-glycol. In one embodiment, the composition of the invention is an emulsion, and the osmolality modifying agent is advantageously comprised in the oil phase of the emulsion. An example of suitable osmolality modifying agent present in the oil phase of the emulsion is glycerol.

In one embodiment of the invention, the amount of osmolality modifying agent in the composition, preferably in the emulsion, ranges from about 0.5% to about 25% in weight to the total weight of the composition, preferably from about 1 to about 10% w/w, more preferably is of about 5% w/w.

In one embodiment of the invention, the amount of osmolality modifying agent in the emulsion ranges from about 10% to about 45% in weight to the total weight of the oily phase of the emulsion, preferably from about 20 to about 35% w/w, more preferably is of about 27% w/w.

In one embodiment, the composition of the invention further comprises a pH buffering agent.

Indeed, preferably, the pH of the composition of the emulsion is constant, and ranges from about 6 to about 8, preferably from about 6.8 to about 7.2, more preferably is of about 7.

Suitable examples of pH buffering agents include, but are not limited to, NaOH, phosphate buffer, citrate buffer, tris buffer, histidine buffer and acetate buffer. Advantageously, the pH buffering agent is NaOH.

In one embodiment, the composition of the invention is an emulsion, and the pH buffering agent is advantageously present in the aqueous phase of the emulsion.

In one embodiment of the invention, the amount of pH buffering agent in the composition, preferably in the emulsion, is sufficient for buffering the composition at the desired pH.

In one embodiment, the amount of pH buffering agent in the composition, preferably in the emulsion, ranges from about 0.1% to about 0.5% in weight to the total weight of the composition, preferably of the emulsion, preferably from about 0.2 to about 0.4% w/w, more preferably is of about 0.29% w/w.

In one embodiment of the invention, the composition is an emulsion, and the amount of pH buffering agent in the emulsion ranges from about 0.2% to about 0.5% in weight to the total weight of the aqueous phase of the emulsion, preferably from about 0.3 to about 0.4% w/w, more preferably is of about 0.35% w/w.

In one embodiment of the invention, the composition of the invention further comprises urea. Urea may help to denature secondary structures in the active phosphorothioate oligonucleotide present in the composition of the invention.

In one embodiment, the composition of the invention is an emulsion, and urea is preferably comprised in the aqueous phase of the emulsion.

In one embodiment, the amount of urea in the composition of the invention ranges from about 0.5% to about 20% in weight to the total weight of the composition, preferably from about 1 to about 10% w/w, more preferably is of about 4% w/w.

In one embodiment, the amount of urea in the emulsion of the invention ranges from about 0.5% to about 20% in weight to the total weight of the aqueous phase of the emulsion of the invention, preferably from about 1 to about 10% w/w, more preferably is of about 4.9% w/w.

In one embodiment, the composition of the invention is an oil-in-water emulsion or a water-in-oil-in-water emulsion, preferably a water-in-oil-in-water emulsion, wherein said emulsion comprises:
- an aqueous phase, wherein the amount of the aqueous phase in the emulsion ranges from about 70 to 99% in weight to the total weight of the emulsion, preferably is of about 81.5% w/w; and
- an oil phase, wherein the amount of the oily phase in the emulsion ranges from about 1 to about 30% in weight to the total weight of the emulsion, preferably is of about 18.5%;
wherein
- the aqueous phase comprises:
   - a phosphorothioate oligonucleotide, preferably in an amount ranging from about 0.01% to about 3% in weight to the total weight of the emulsion, preferably from about 0.04% to about 2% w/w and more preferably is of about 1.72% w/w; or in an amount ranging from about 0.01% to about 3% in weight to the total weight of the aqueous phase of the emulsion, preferably from about 0.04% to about 2.5% w/w and more preferably is of about 2.11% w/w; and
   - an agent comprising a group thiol, preferably in an amount ranging from about 0.5% to about 5% in weight to the total weight of the composition, preferably from about 1 to about 3% w/w, more preferably is of about 2% w/w; or in an amount ranging from about 0.5% to about 5% in weight to the total weight of the aqueous phase of the emulsion of the invention, preferably from about 1 to about 3% w/w, more preferably is of about 2.45% w/w; and
   - optionally, a viscosity modifying agent, preferably Carbopol® 980 NF; preferably in an amount ranging from about 0.01 to about 0.1% in weight to the total weight of the emulsion, more preferably of about 0.05% w/w, or ranging from about 0.01% to about 0.2% in weight to the weight of the aqueous phase, preferably of about 0.061% w/w; and
   - optionally, a pH buffering agent, preferably NaOH, in an amount sufficient for providing a pH ranging from about 6 to about 8, preferably from about 6.8 to about 7.2, more preferably of about 7; such as, for example, in an amount ranging from about 0.1 to about 0.5% in weight to the total weight of the emulsion, preferably of about 0.29% w/w, or ranging from about 0.2% to about 0.5% in weight to the weight of the aqueous phase, preferably of about 0.35% w/w; and
   - optionally, urea, in an amount ranging from about 0.5 to about 20% in weight to the total weight of the emulsion, preferably of about 4% w/w, or ranging from about 0.5% to about 20% in weight to the weight of the aqueous phase, preferably of about 4.9% w/w; and
- the oil phase comprises:
   - an oil, preferably MCT, more preferably Miglyol 812N, preferably in an amount ranging from about 1 to about 20% in weight to the total weight of the emulsion, preferably of about 8% w/w, or ranging from about 25% to about 75% in weight to the weight of the oil phase, preferably of about 43.2% w/w; and
   - optionally, at least one emulsifying agent, preferably a mixture of glyceryl stearate and of PEG-75; preferably in an amount ranging from about 1 to about 10% in weight to the total weight of the emulsion, preferably of about 3.5% w/w, or ranging from about 5% to about 40% in weight to the weight of the oil phase, preferably of about 18.9% w/w; and
   - optionally a thickening agent, preferably cetyl alcohol; preferably in an amount ranging from about 0.1 to about 10% in weight to the total weight of the emulsion, preferably of about 2% w/w, or ranging from about 1% to about 30% in weight to the weight of the oil phase, preferably of about 10.8% w/w; and
   - optionally, an osmolality modifying agent, preferably glycerol; preferably in an amount ranging from about 0.5 to about 25% in weight to the total weight of the emulsion, preferably of about 5% w/w, or ranging from about 10% to about 45% in weight to the weight of the oil phase, preferably of about 27% w/w.

Also included within the scope of this invention are preserved compounds which increase in viscosity upon administration to the eye. Examples of such compounds are "gelling polysaccharides" are disclosed in US 5,212,162, which is incorporated in its entirety herein by reference. Also disclosed in this patent are ophthalmic formulations containing carrageenans and furcellarans which are administered as partially gelled liquids which gel upon instillation into the eye. Additionally, US 4,136,173, US 4,136,177, and US 4,136,178, disclose the use of therapeutic compositions containing xanthan gum and locust bean gum which are delivered in liquid form to the eye and which gel upon instillation. US 4,861,760 discloses ophthalmological compositions containing gellan gum which are administered to the eye as non-gelled liquids and which gel upon instillation. Each of these four patents is incorporated in its entirety herein by reference. Also within the scope of this invention are preserved oils, ointments, gels and the like.

In one embodiment, the phosphorothioate oligonucleotide is stable in the composition of the invention. In other words, in one embodiment, the composition of the invention prevents degradation of the phosphorothioate oligonucleotide.

The stability of the phosphorothioate oligonucleotide can be assessed, after extraction of the oligonucleotide from the composition, by ionic chromatography (HPLC) coupled to UV detection. Examples of chromatographic conditions that may be used include, but are not limited to, the following conditions:
- pre-columns and columns: DIONEX DNAS Pack pa-100
- mobile phases:
   o A: NaOH 10 mM in water
   o B: NaOH 10 mM and NaClO₄ 0.375 M.

In one embodiment of the invention, the phosphorothioate oligonucleotide is stable for at least about 1 day, preferably at least about 1 week, more preferably for at least about 1 month or more at room temperature (i.e. a temperature ranging from about 15 to about 25°C, preferably at a temperature of about 20°C) in the composition of the invention.

In another embodiment of the invention, the phosphorothioate oligonucleotide is stable for at least about 10 min, preferably at least about 1 hour, more preferably for at least about 5, 6, 7, 8, 9, 10 hours or more at a temperature of at least about 40°C, preferably of at least about 50°C, more preferably of about 60°C.

The present invention further relates to a pharmaceutical composition comprising the composition of the invention and at least one pharmaceutically acceptable excipient.

The present invention further relates to a medicament comprising the composition of the invention.

The pharmaceutical composition of the invention may be formulated into a variety of topically or injectable administrable compositions. Examples of such formulations include, but are not limited to, emulsions, gels, ointments, micelles or eye drops.

In one embodiment, the composition is formulated in accordance with routine procedures as a pharmaceutical composition adapted for topical administration (in particular for topical administration to the eye) or for injection (in particular for subcunjunctival injection, injection into the conjunctival sac, or intravitreal injection) to human beings.

In one embodiment of the invention, said composition, pharmaceutical composition or medicament of the invention is packaged in the form of unit dose. Examples of unit doses that may be used include, but are not limited to, a container capable of dispensing eye drops such as common manual bulb-operated pipette or small squeeze bottle with a dropper tip; a container to which a device for the placement of eye drops may be applied; a container capable of atomizing drops or droplets and a disposable syringe.

It will be understood that the total daily usage of the composition, pharmaceutical composition and medicament of the present invention will be decided by the attending physician within the scope of sound medical judgment. The specific therapeutically effective amount for any particular patient will depend upon a variety of factors including the disorder being treated and the severity of the disorder; activity of the specific phosphorothioate oligonucleotide employed; the specific composition employed, the age, body weight, general health, sex and diet of the subject; the time of administration, route of administration, and rate of excretion of the specific phosphorothioate oligonucleotide employed; the duration of the treatment; drugs used in combination or coincidental with the specific phosphorothioate oligonucleotide employed; and like factors well known in the medical arts. For example, it is well within the skill of the art to start doses of a therapeutic compound at levels lower than those required to achieve the desired therapeutic effect and to gradually increase the dosage until the desired effect is achieved.

The present invention further relates to the composition, pharmaceutical composition or medicament of the invention for treating or for use in the treatment of an angiogenic disorder in a subject in need thereof, wherein the composition, pharmaceutical composition or medicament preferably comprises an IRS-1 inhibitor as described hereinabove.

Angiogenesis is a fundamental process by means of which new blood vessels are formed. Angiogenesis is essential in multiple normal physiological phenomena such as reproduction, development and even wound healing. Angiogenic disorder refers to a pathological neovascularization as is occurring in a number of diseases, where the pathological neovascularization is linked to the invasion of tissues and organs by neovessels. Examples of angiogenic disorders include, but are not limited to, ocular neovascular diseases (such as, for example, ischemic retinopathy, diabetic retinopathy, retinopathy of prematurity, retinal vein occlusions, age-related macular degeneration, corneal neovascularisation, neovascular glaucoma), atherosclerosis, arthritis, psoriasis, obesity, cancer and Alzheimer's disease. In one embodiment of the invention, the angiogenic disorder is an ocular angiogenic disease.

According to an embodiment, the ocular angiogenic disease is associated with retinal, peripheral retinal and/or choroidal neovascularization. Examples of such angiogenic diseases include, but are not limited to uveitis, choroiditis, choroidal vasculopathy, hypersensitive retinopathy, retinochoroiditis, chorioretinitis, retinal angiomatosis, retinal degeneration, macular degeneration, AMD, retinal detachment, retinal neovascularisation, proliferative vitreoretinopathy, retinopathy of prematurity (ROP), central serous chorioretinopathy, diabetic retinopathy, posterior segment trauma, retinal vascular pathologies, retinal telangiectesa, endophthalmitis, macular edema, radiation-induced retinopathy, cystoid macular edema, diabetic retinopathy, inflammatory pathologies of the retina, sickle cell anemia, sickle cell retinopathy, sarcoid, syphilis, pseudoxanthoma elasticum, Pagets disease, vein occlusion, artery occlusion, carotid obstructive disease, chronic uveitis/vitritis, mycobacterial infections, Lyme's disease, systemic lupus erythematosis, systemic pathologies with implications for the retina, Eales disease, Bechets disease, infections causing a retinitis or choroiditis, presumed ocular histoplasmosis, Bests disease, myopia, optic pits, Stargarts disease, pars planitis, chronic retinal detachment, hyperviscosity syndromes, toxoplasmosis, trauma and post-laser complications.

According to an embodiment, the ocular angiogenic disease is associated with corneal neovascularization. Examples of such angiogenic diseases include, but are not limited to diabetic retinopathy, retinopathy of prematurity, corneal graft rejection, neovascular glaucoma and retrolental fibroplasia, epidemic keratoconjunctivitis, Vitamin A deficiency, contact lens overwear, atopic keratitis, superior limbic keratitis, pterygium keratitis sicca, sjogrens, acne rosacea, phylectenulosis, syphilis, Mycobacteria infections, lipid degeneration, chemical bums, bacterial ulcers, fungal ulcers, Herpes simplex infections, Herpes zoster infections, protozoan infections, Kaposi sarcoma, Mooren ulcer, Terrien's marginal degeneration, mariginal keratolysis, trauma, rheumatoid arthritis, systemic lupus, polyarteritis, Wegeners sarcoidosis, Scleritis, Steven's Johnson disease, periphigoid radial keratotomy, and comeal graph rejection.

According to an embodiment, the ocular angiogenic disease is selected from the group comprising diseases associated with rubeosis (neovascularization of the angle) and diseases caused by the abnormal proliferation of fibrovascular or fibrous tissue including all forms of proliferative vitreoretinopathy, whether or not associated with diabetes.

The present invention further relates to a method for treating an angiogenic disorder in a subject in need thereof, wherein the method comprises administering to the subject the composition, pharmaceutical composition or medicament of the invention, wherein said composition, pharmaceutical composition or medicament preferably comprises an IRS-1 inhibitor as described hereinabove.

The present invention further relates to a method for inhibiting angiogenesis in a subject in need thereof, wherein the method comprises administering to the subject the composition, pharmaceutical composition or medicament of the invention, wherein said composition, pharmaceutical composition or medicament preferably comprises an IRS-1 inhibitor as described hereinabove.

The present invention further relates to a method for preventing, stabilizing and/or inhibiting lymph of blood vascularization or corneal angiogenesis in a subject in need thereof, wherein the method comprises administering to the subject the composition, pharmaceutical composition or medicament of the invention, wherein said composition, pharmaceutical composition or medicament preferably comprises an IRS-1 inhibitor as described hereinabove.

The present invention further relates to a method for preventing or stabilizing neovascularization in a subject in need thereof, wherein the method comprises administering to the subject the composition, pharmaceutical composition or medicament of the invention, wherein said composition, pharmaceutical composition or medicament preferably comprises an IRS-1 inhibitor as described hereinabove.

In one embodiment, a therapeutically effective amount of the composition, pharmaceutical composition or medicament of the invention is administered to the subject.

In one embodiment of the invention, the amount of said phosphorothioate antisense oligonucleotide inhibitor of IRS-1 to be administrated per eye per day ranges from about 8 to about 40 µg, preferably from about 10 to about 35 µg, preferably from about 12 to about 30 µg, more preferably from about 14 to about 25 µg and even more preferably from about 16 to about 20 µg.

In one embodiment of the invention, the composition, pharmaceutical composition or medicament of the invention is to be administrated as drops of about 1 µl to about 1ml, preferably from about 10 µl to about 100 µl, more preferably of about 50 µl per eye. It is generally acknowledged that, when a pharmaceutical composition is administrated in the form of an eye drop i.e. 50 µl, only about 10 µl may stay on the eye.

In one embodiment, one or two drops of the composition of the invention are administered per eye per day.

In one embodiment of the invention, said pharmaceutical composition is to be administrated once, twice, three or more times a day. In one embodiment, said pharmaceutical composition is to be administrated once a day. In another embodiment, said pharmaceutical composition is to be administrated twice a day, preferably in the morning and in the evening.

In one embodiment of the invention, the amount of the phosphorothioate oligonucleotide, preferably of the IRS-1 antisense, to be administrated per eye per day ranges from about 20 to 100 µg, preferably from about 30 to about 90 µg, more preferably from about 40 to about 90 µg, even more preferably from about 50 to about 90 µg, still even more preferably from about 60 to about 90 µg, still even more preferably from about 70 to about 90 µg and still even more preferably from about 80 to about 90 µg.

In one embodiment, the amount of the phosphorothioate oligonucleotide, preferably of the IRS-1 antisense, to be administrated per eye per day ranges from about 40 to about 50 µg, preferably is of about 43 µg: corresponding for example, to about 20 to 25 µg per drop with an administration of 2 drops per eye per day.

In another embodiment, the amount of the phosphorothioate oligonucleotide, preferably of the IRS-1 antisense, to be administrated per eye per day ranges from about 80 to about 100 µg, preferably is about 86 µg: corresponding for example, to about 40 to 50 µg per drop with an administration of 2 drops per eye per day.

In said embodiment, the pharmaceutical composition is preferably in the form of a unit dose for administering from about 80 to 100 µg, preferably about 86 µg, of said phosphorothioate oligonucleotide per eye per day.

For example, two drops of 50 µl of a composition comprising 0.80 to 1 mg/ml of said antisense oligonucleotide may be administrated per eye in one time to the subject in need thereof. In another example, one drop of 50 µl of a composition comprising 1.60 to 2 mg/ml of said antisense oligonucleotide may be administrated per eye to the subject in need thereof.

In another example, one drop of 50 µl of a composition comprising 0.80 to 1 mg/ml of said phosphorothioate oligonucleotide is administrated per eye twice a day to the subject in need thereof.

The present invention also relates to a method for obtaining a sterile composition comprising at least one fatty acid and/or at least one emulsifying agent, a phosphorothioate oligonucleotide and an agent comprising a thiol group, wherein preferably said phosphorothioate oligonucleotide is stable within the sterile composition.

The method of the invention comprises the following steps:
- mixing all the components of the composition including the at least one fatty acid and/or at least one emulsifying agent and the agent comprising the thiol group; and
- sterilizing the composition resulting from the previous step by steam sterilization, i.e. heating in an autoclave; and
- adding the phosphorothioate oligonucleotide to the autoclaved composition under sterile conditions.

Preferably, the phosphorothioate oligonucleotide was previously sterilized before addition to the autoclaved composition, preferably by filtration, more preferably using a filter with a size ranging from about 0.2 to about 0.8 µm, preferably from about 0.4 to about 0.5 µm, and more preferably through a 0.45 µm filter.

Examples of agents comprising a thiol group is selected from the group comprising DL-cysteine, N-acetylcysteine, lipoic acid, glutathione, 2-mercapto-5-benzimidazole salts, 2-mercaptoethanesulfonic acid salts. Preferably, the agent comprising a thiol group is selected from the group comprising DL-cysteine, N-acetylcysteine and lipoic acid.

In one embodiment, the sterile composition is an emulsion such as a water-in-oil emulsion, an oil-in-water emulsion or a water-in-oil-in-water emulsion. Preferably the sterile composition obtained is a water-in-oil-in-water emulsion.

The present invention further relates to a composition obtained by, or obtainable by, the method of the invention.

In one embodiment, the method of the invention is for preparing a composition, preferably an emulsion, of the invention as described hereinabove.

The stability of said phosphorothioate oligonucleotide comprised in the composition obtained by the method of the invention can be assessed, after extraction of the oligonucleotide from the emulsion, by ionic chromatography (HPLC) coupled to UV detection.

In one embodiment of the emulsion obtained by the method of the invention, the phosphorothioate oligonucleotide is stable for at least about 1 day, preferably at least about 1 week, more preferably for at least about 1 month or more at room temperature (i.e. a temperature ranging from about 15 and about 25°C, preferably at a temperature of about 20°C) in the composition of the invention.

In another embodiment of the emulsion obtained by the method of the invention, the phosphorothioate oligonucleotide is stable for at least about 10 min, preferably at least about 1 hour, more preferably for at least about 5, 6, 7, 8, 9, 10 hours or more at a temperature of at least about 40°C, preferably of at least about 50°C, more preferably of about 60°C.

In one embodiment, the composition to be obtained by the method of the invention is an emulsion, preferably a water-in-oil-in-water emulsion and the components to be mixed in the first step of the method of the invention are:
- in the aqueous phase:
   - an agent comprising a thiol group, preferably in an amount ranging from about 0.5% to about 5% in weight to the total weight of the composition, preferably from about 1 to about 3% w/w, more preferably is of about 2% w/w; or in an amount ranging from about 0.5% to about 5% in weight to the total weight of the aqueous phase of the emulsion of the invention, preferably from about 1 to about 3% w/w, more preferably is of about 2.45% w/w;
   - optionally, a viscosity modifying agent, preferably Carbopol® 980 NF; in an amount ranging from about 0.01 to about 0.1, preferably of about 0.05% in weight to the total weight of the emulsion, or ranging from about 0.01% to about 0.2%, preferably of about 0.061% in weight to the weight of the aqueous phase; and
   - optionally, a pH buffering agent, preferably NaOH, in an amount ranging from about 0.1 to about 0.5, preferably of about 0.29% in weight to the total weight of the emulsion, or ranging from about 0.2% to about 0.5%, preferably of about 0.35% in weight to the weight of the aqueous phase; and
   - optionally, urea, in an amount ranging from about 0.5 to about 20, preferably of about 4% in weight to the total weight of the emulsion, or ranging from about 0.5% to about 20%, preferably of about 4.9% in weight to the weight of the aqueous phase; and
- in the oily phase:
   - an oil, preferably MCT, more preferably Miglyol 812N, in an amount ranging from about 1 to about 20, preferably of about 8% in weight to the total weight of the emulsion, or ranging from about 25% to about 75%, preferably of about 43.2% in weight to the weight of the oily phase; and
   - optionally, at least one emulsifying agent, preferably a mixture of glyceryl stearate and of PEG-75; in an amount ranging from about 1 to about 10, preferably of about 3.5% in weight to the total weight of the emulsion, or ranging from about 5% to about 40%, preferably of about 18.9% in weight to the weight of the oily phase; and
   - optionally a thickening agent, preferably cetyl alcohol; in an amount ranging from about 0.1 to about 10, preferably of about 2% in weight to the total weight of the emulsion, or ranging from about 1% to about 30%, preferably of about 10.8% in weight to the weight of the oily phase; and
   - optionally, an osmolality modifying agent, preferably glycerol; in an amount ranging from about 0.5 to about 25, preferably of about 5% in weight to the total weight of the emulsion, or ranging from about 10% to about 45%, preferably of about 27% in weight to the weight of the oily phase.

In one embodiment, the method of the invention comprises (i) mixing the ingredients of the aqueous phase, (ii) mixing the ingredients of the oil phase, and (iii) mixing the oil and aqueous phases.

In one embodiment, the mixing step(s) of the method of the invention is/are carried out by magnetic stirring.

In one embodiment, the autoclaving step comprises heating the composition, preferably the emulsion, at a temperature ranging from about 100°C to about 130°C, preferably at a temperature ranging from about 116°C to about 125°C and more preferably at about 121°C; for a period ranging from about 10 to about 30 minutes, preferably from about 15 to about 25 minutes and more preferable of about 20 minutes. Preferably, the autoclaving step comprises heating the composition, preferably the emulsion, at about 121°C for about 20 minutes. In one embodiment, the autoclaving step is carried out with a pressure ranging from about 0.5 to about 1.5 bar, preferably from about 0.8 to about 1.2 bar, and more preferably of about 1 bar.

The present invention further relates to a method for preventing and/or inhibiting the degradation of a phosphorothioate oligonucleotide in a composition comprising at least one fatty acid and/or at least one emulsifying agent and subjected to steam sterilization (i.e. heated in an autoclave), wherein said method comprises adding an agent comprising a thiol group within the composition before the step of steam sterilization.

In one embodiment, the degradation of the phosphorothioate oligonucleotide comprises sequential oxidation provoked by the attack of highly reactive entities liberated from the at least one fatty acid and/or from the at least one emulsifying agent due to the high temperature of steam sterilization, and subsequent β-elimination.

### BRIEF DESCRIPTION OF THE DRAWINGS

**Figure 1** is a graph showing a representative chromatogram of a standard solution of the phosphorothioate oligonucleotide GS-101 analyzed by ionic chromatography.
**Figure 2** is a graph showing the chromatogram of GS-101 extracted from an autoclaved emulsion after 1 day incubation at room temperature.
**Figure 3** is a graph showing the chromatogram of GS-101 extracted from an autoclaved emulsion after 14 days incubation at room temperature.
**Figure 4** is a combination of graphs showing the chromatograms of GS-101 extracted from an autoclaved emulsion after incubation at 60°C (A) for 2 hours and (B) for 6 hours.
**Figure 5** is a combination of graphs showing the chromatogram of GS-101 extracted from an autoclaved emulsion comprising an antioxidant after 1 day incubation at room temperature. The antioxidant was added after the autoclaving step during the preparation of the emulsion: (A) addition of vitamin E, (B) addition of lipoic acid, (C) addition of DL-cysteine and (D) addition of N-actylcysteine.
**Figure 6** is a graph showing the chromatogram of GS-101 extracted from an autoclaved emulsion comprising DL-cysteine after 30 days incubation at room temperature. DL-cysteine, an agent comprising a thiol group, was added before the autoclaving step.
**Figure 7** is a combination of graphs showing the chromatogram of GS-101 extracted from an autoclaved emulsion comprising an antioxidant after 6 hours incubation at 60°C. The antioxidant was added before the autoclaving step: (A) addition of N-acetylcysteine which comprises a thiol group and (B) addition of vitamin E which does not comprise a thiol group.
**Figure 8** is a scheme showing the sequential peripheral oxidation of phosphorothioate derived oligonucleotide.
**Figure 9** is a scheme showing the sequential peripheral oxidation of phosphorothioate derived oligonucleotide followed by β-elimination reaction.

### EXAMPLES

The present invention is further illustrated by the following examples.

### Example 1: Preparation of sterile GS-101 emulsions

### Preparation of the GS-101 standard solution

The solution was prepared by dissolving 86 mg in 10 ml of distilled water of the phosphorothioate antisense oligonucleotide GS-101 having the sequence SEQ ID NO: 2 (5'-TCTCCGGAGGGCTCGCCATGCTGCT-3'). The solution was filtered through 0.45 µm filter, distributed to Eppendorf tubes (0.2 ml/tube) and stored at -20°C.

### Preparation of the aqueous phase

50 mg of Carbopol 980NF were added to 85 ml of distilled water in a 100-ml beaker. The mixture was solubilized under magnetic stirring for at least 30 min and the pH was adjusted to 7 with 1 N NaOH solution. The resulting aqueous solution was then heated to 70°C.

### Preparation of the oil phase

8 g of Miglyol 812N, 3.5 g of Gelot 64 and 2 g of cetyl alcohol were added in a 100-ml beaker. The beaker was then placed onto a magnetic stirrer-heater adjusted at 70°C. The mixture was solubilized and homogenized under continuous stirring (300 tour/min) at 70°C for 10 min.

### Preparation of the bulk emulsion

The prepared oil phase kept at 70°C and the prepared aqueous phase kept at 70°C were mixed together at 70°C to form the bulk ophthalmic emulsion. The resulting bulk emulsion was kept under magnetic stirring (300 tour/min) for 10 min at 70°C. The resulting emulsion was then transferred into a bottle (500 ml) for autoclaving.

### Sterilization of the bulk emulsion

The bulk ophthalmic emulsion (without GS-101) was autoclaved for 20 min at 121°C. The emulsion was kept under agitation until its cooling to room temperature.

### Preparation of the GS-101 sterile emulsion

Under sterile conditions, the emulsion was distributed to Eppendorf tubes (1-1.2 g/tube). One hundred microliter (100 µl) of the filtered solution of GS-101 was then added to each tube and mixed under sterile conditions.

### Example 2: Stability of GS-101 in the emulsions of the invention

### Materials and Methods

### Incubation of the emulsion

The bulk emulsion was prepared, sterilized, cooled to room temperature and enriched with GS-101 as described in Example 1. The resulting emulsions were incubated at room temperature or 60°C for different time periods.

### Extraction of GS-101

2 ml of N-Hexane were added to 2 ml GS-101 enriched emulsion using a glass pipette and manually mixed for 1 minute. The tube was centrifuged at 3,000 g for 10 min at 10°C. At the end of the centrifugation, three layers were obtained: the upper hexane layer, the middle lipid ring, and the lower aqueous layer containing GS-101. The lower aqueous layer containing GS-101 was collected using a 21G needle mounted to a syringe. This aqueous solution was transferred into another tube and centrifuged at 16,000 g for 5 min to further isolate the GS-101 containing aqueous fraction from lipid contamination. The aqueous phase was then collected using a 21G needle. This aqueous solution was subjected to analysis.

### Analysis by ionic chromatograply (HPLC) coupled to UV detection

| | |
|---|---|
| Pre-column | DIONEX DNA Pack Ref 43018 50*4 mm 25 µm pa-100 |
| Column | DIONEX DNA Pack Ref 43010 250*4 mm 25 µm pa-100 |
| Mobile phase A | NaOH 10 mM in water |
| Mobile phase B | NaOH 10 mM and NaCl0₄ 0.375 M |

### Gradient

| **Time (min)** | **% A** | **% B** |
|---|---|---|
| 0 | 50 | 50 |
| 21 | 0 | 100 |
| 25 | 0 | 100 |
| 30 | 50 | 50 |
| 35 | 50 | 50 |

| | |
|---|---|
| Flow rate | 1.5 ml/min |
| Run time | 35 min |
| Injection volume | 20 µl |
| Column temperature | 40°C |
| Detection wavelength | 259 nm. |

### Results

### In a standard emulsion GS-101 is rapidly degraded

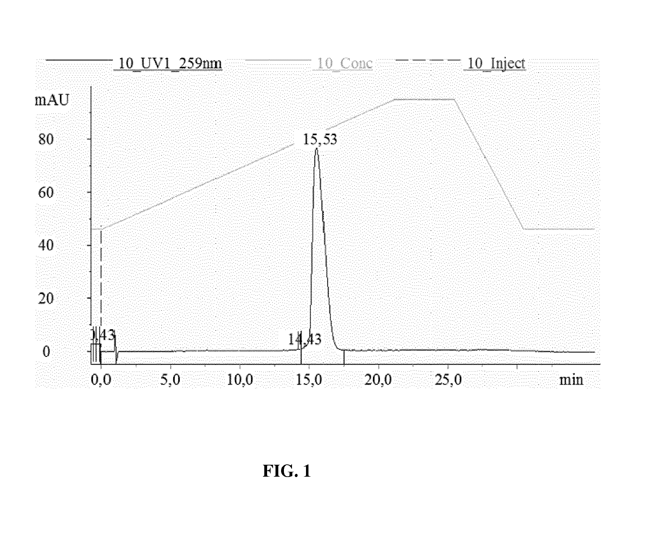

Before starting the analysis of GS-101 samples, controls were carried out with the analysis by chromatography of distilled water and of a standard solution of GS-101. A stable base line was obtained following injection of distilled water. The injection of a standard solution of GS-101 revealed that the retention time of the peak of GS-101 was stable with no significant variability between three analyses as illustrated in the Table 1 below. Table 1 shows the retention time, area and height of the GS-101 peak of each of the three analyses. **Figure 1** shows a representative chromatogram of the standard stable aqueous solution of GS-101.

**Table 1**

| | Retention Time (min) | Area (mAU*min) |
|---|---|---|
| Analysis 1 | 15.38 | 74.8476 |
| Analysis 2 | 15.53 | 72.8236 |
| Analysis 3 | 15.37 | 73.7512 |

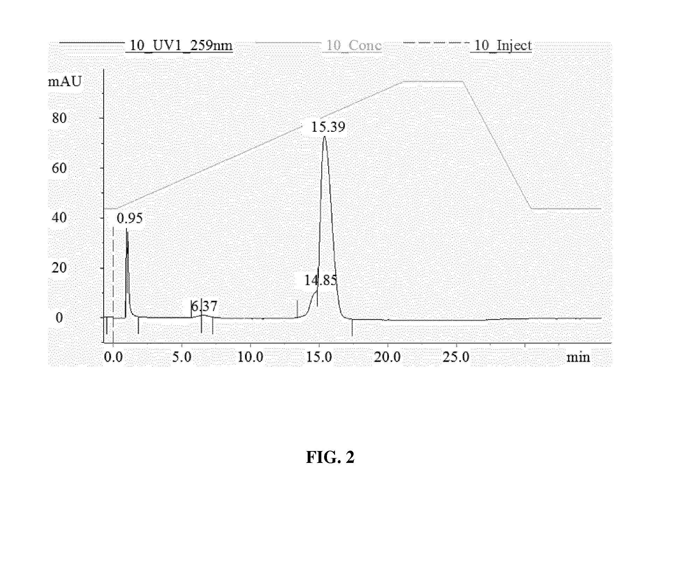

The autoclaved emulsion enriched with GS-101 was incubated for 1, 2, 7 and 14 days at room temperature. GS-101 was then extracted and analyzed by chromatography. **Figure 2** shows that after 1 day, a smaller peak precedes the standard peak of GS-101 indicating that degradation of GS-101 occurred during the incubation. The height and area of the GS-101 peaks are listed in table 2. Reporting the area of the smaller peak (6.5642 mAU*min) to that of the 2 GS-101 peaks combined (75.4013 mAU*min) showed that more than 8% of the GS-101 incorporated in the emulsion was degraded.

**Table 2**

| Retention Time (min) | Area (mAU*min) |
|---|---|
| 14.85 | 6.5642 |
| 15.39 | 68.8371 |

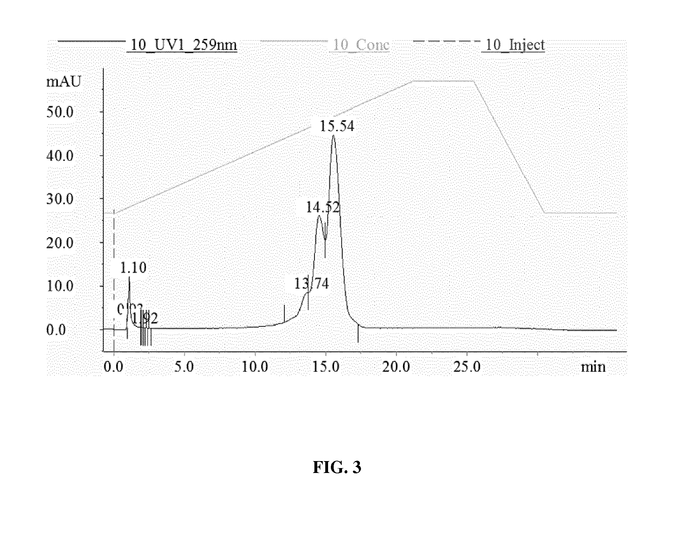

**Figure 3** shows that after 14 days at room temperature, further degradation of GS-101 occurred as 2 smaller peaks precede the standard GS-101 peak. The height and area of the GS-101 peaks are listed in table 3. Similarly reporting their combined area (24.0909 mAU*min) to the total GS-101 area (65.4598 mAU*min) indicated that more than 35% of the GS-101 was degraded.

**Table 3**

| Retention Time (min) | Area (mAU*min) |
|---|---|
| 13.74 | 3.9635 |
| 14.52 | 20.1274 |
| 15.54 | 41.3689 |

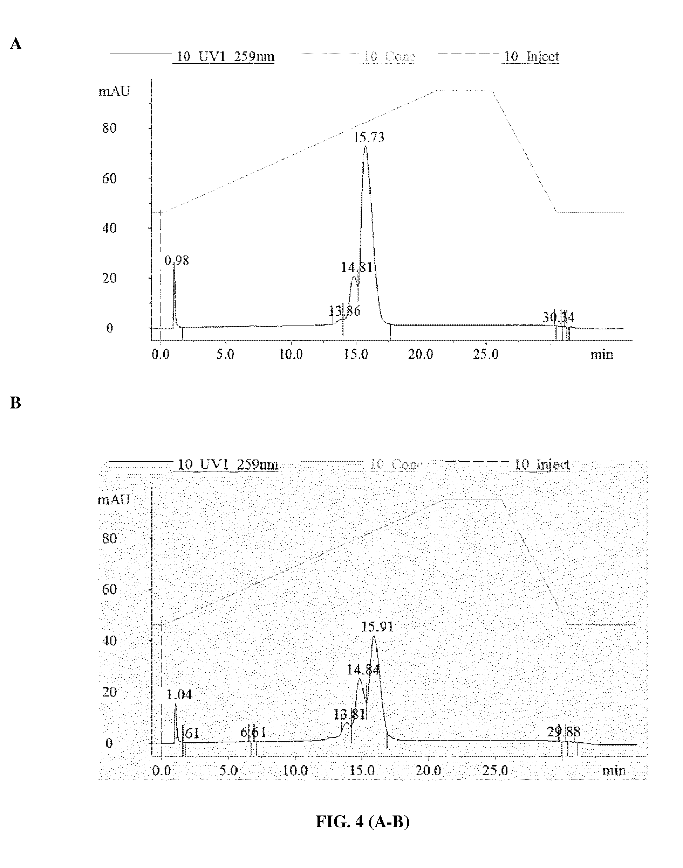

Furthermore the autoclaved emulsion enriched with GS-101 was also incubated at 60°C for 2h and 6h. GS-101 was then extracted and analyzed by chromatography. **Figure 4A** shows that after 2h of incubation 16% of the GS-101 was already degraded. The degradation increased to 32% after 6h at 60°C as shown in **Figure 4B****.** The height and area of the GS-101 peaks obtained in both conditions are listed in table 4.

**Table 4**

| | Retention Time (min) | Area (mAU*min) |
|---|---|---|
| 2 hours at 60°C | 13.86 | 0.8634 |
| | 14.81 | 12.5045 |
| | 15.73 | 68.5381 |
| 6 hours at 60°C | 13.81 | 1.1757 |
| | 14.84 | 12.6498 |
| | 15.91 | 29.0388 |

The degradation of GS-101 is much faster at 60°C than at room temperature suggesting it occurs through an oxidative mechanism, as it is well established that high temperatures could generate highly reactive chemical entities and/or free radicals.

### Addition of antioxidants to the standard emulsion after steam sterilization does not protect GS-101 from degradation

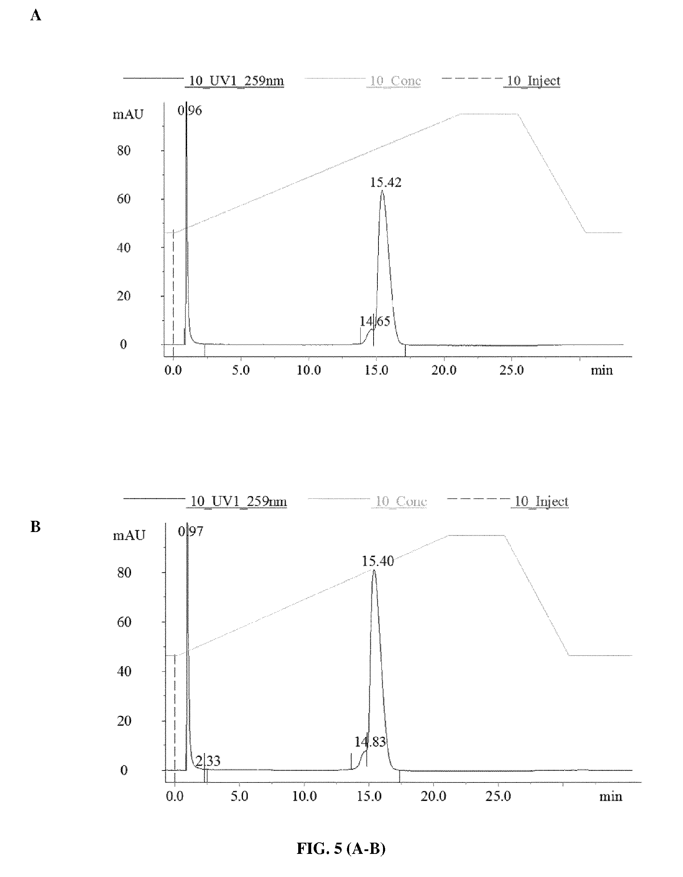

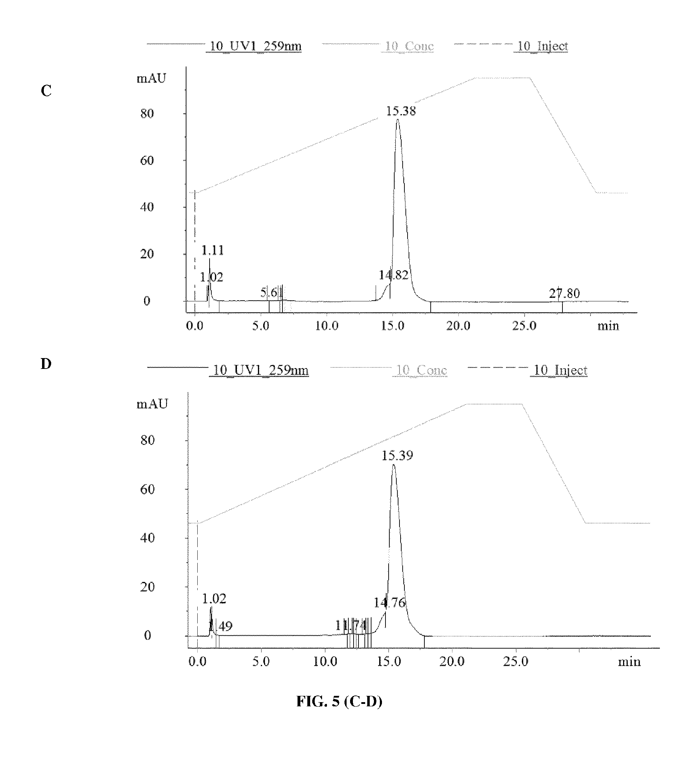

To prevent any degradation of GS-101 through an oxidative mechanism, antioxidants (vitamin E, lipoic acid, DL-cysteine or N-acetylcysteine) were added at a 2% final concentration to the emulsion after its steam sterilization by autoclaving. The antioxidants were thus added just before the addition of GS-101. The resulting emulsions were incubated at room temperature for 1, 3 and 7 days. GS-101 was then extracted from the different emulsions and analyzed by chromatography. After 1 day incubation, degradation of GS-101 had already occurred within the 4 emulsions as observed with the appearance of a smaller GS-101 peak preceding the standard GS-101 peak on the chromatograms of **Figure 5****.** The height and area of the GS-101 peaks are listed in table 5 below. Reporting the area of the smaller peak to the combined areas of the 2 GS-101 peaks indicated that around 5% of the GS-101 was degraded with all antioxidants tested. None of the antioxidants had a significant effect in protecting the GS-101 from degradation.

**Table 5**

| | Retention Time (min) | Area (mAU*min) |
|---|---|---|
| Vitamin E | 14.65 | 3.2756 |
| | 15.42 | 58.5941 |
| Lipoic acid | 14.83 | 4.3382 |
| | 15.40 | 76.2065 |
| DL-cysteine | 14.82 | 3.9340 |
| | 15.38 | 75.6551 |
| N-acetylcysteine | 14.76 | 4.8207 |
| | 15.39 | 72.4214 |

These results suggest that an oxidative mechanism alone cannot account for the degradation of GS-101. The different chromatograms show that degradation of GS-101 in the emulsion started by the appearance of high molecular weight derivatives of GS-101 (i.e. n-1 to n-3) (peaks at about 14.5 min in **Figures 2** **and** **5**) and as the time of the incubation increases there is a gradual rise in the proportion of these high molecular weight derivatives of GS-101 (i.e. n-1 to n-3) accompanied with lower molecular weight derivatives of GS-101 (i.e. n-4 to n-7) (peaks at about 13.74 min in **Figure 3** and at about 13.8 min in **Figure 4**). This hydrolytic cascade strongly suggests a gradual and sequential degradation of GS-101 which takes place at the two extremities of the molecule, such as a reaction of β-elimination.

### Steam sterilization after the addition of an agent comprising a thiol group to the emulsion protects GS-101 from degradation

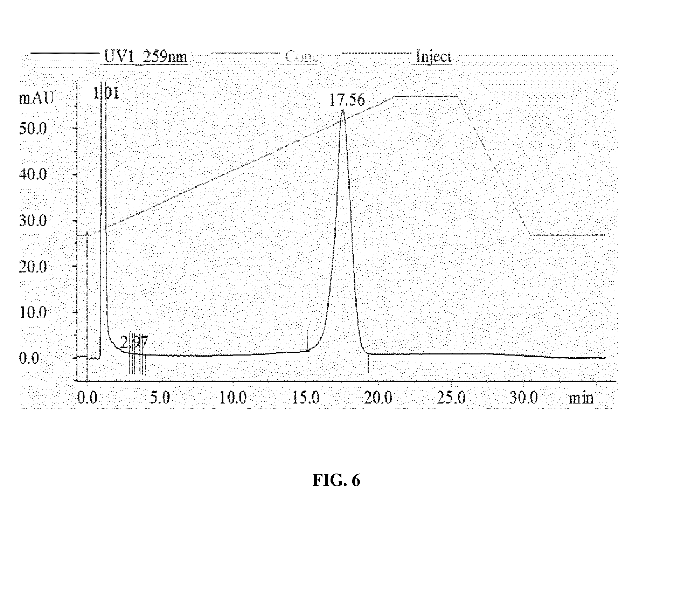

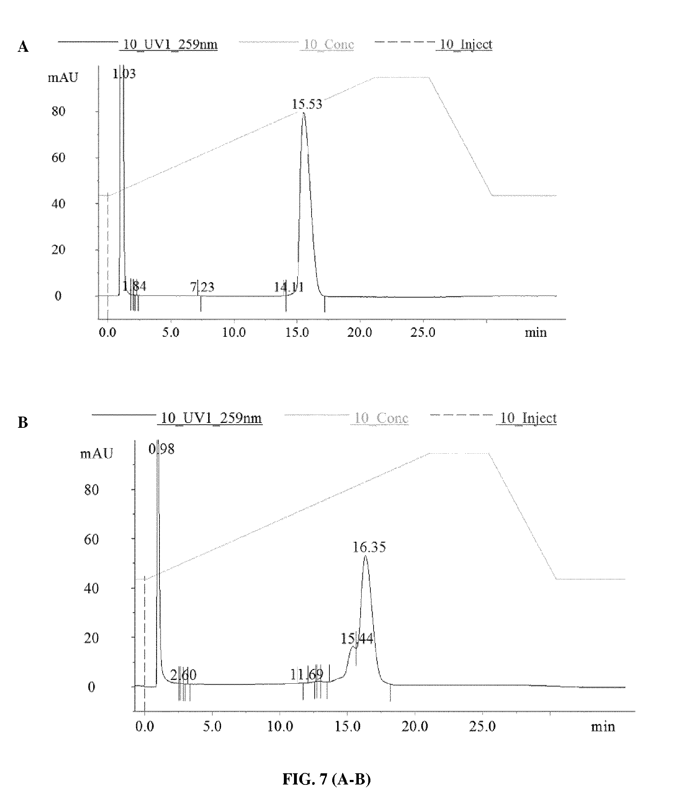

To increase GS-101 stability, agents comprising a thiol group that could compete with the thiol group of GS-101 and inhibit its degradation (lipoic acid, DL-cysteine or N-acetylcysteine) were added to the emulsion at a final concentration of 2% before the steam sterilization by autoclaving. Thus they were added to the emulsion before the sterilization process could induce the generation of highly reactive chemical entities. As a control, vitamin E was also added to the emulsion at a final concentration of 2% before its sterilization. The resulting emulsions were then autoclaved, cooled to room temperature and enriched with GS-101 under sterile conditions. Finally the sterile GS-101 emulsions were incubated at room temperature for 1 to 30 days or at 60° for 1 to 6 hours. GS-101 was extracted from the different emulsions and analyzed by chromatography. **Figure 6** shows that even after 30 days of incubation at room temperature, only the standard peak of GS-101 was observed when GS-101 was extracted from an emulsion in which DL-cysteine was added before the sterilization. Moreover, **Figure 7A** shows that even after 6 hours incubation at 60°C, only the standard peak of GS-101 was observed when GS-101 was extracted from an emulsion in which N-acetylcysteine was added before the sterilization. **Figure 7B** shows that after 6 hours incubation at 60°C, 2 smaller peaks preceding the standard peak of GS-101 were observed when GS-101 was extracted from an emulsion in which vitamin E was added before the sterilization. Addition of vitamin E before the autoclaving step did not protect GS-101 from degradation.

These results demonstrates that the addition of an agent comprising a thiol group before the steam sterilization protects the oligonucleotide from degradation and thus leads to the production of a sterile emulsion wherein the phosphorothioate oligonucleotide is stable.

## Claims

1. A composition comprising a phosphorothioate oligonucleotide and at least one fatty acid and/or at least one emulsifying agent, wherein said composition is sterile and wherein said composition comprises an agent comprising a thiol group, preferably said composition is an ophthalmic composition.

2. The composition according to claim **1,** wherein the composition is an emulsion, preferably an oil-in-water emulsion or a water-in-oil-in-water emulsion.

3. The composition according to claim **1** or claim **2,** wherein the agent comprising a thiol group is selected from the group comprising lipoic acid, DL-cysteine, N-acetylcysteine, glutathione, 2-mercapto-5-benzimidazole salts and 2-mercaptoethanesulfonic acid salts, preferably the agent comprising a thiol group is lipoic acid, DL-cysteine or N-acetylcysteine.

4. The composition according to any one of claims **1** to **3,** wherein all phosphodiester linkages of the phosphorothioate oligonucleotide are replaced by phosphorothioate linkages.

5. The composition according to any one of claims **1** to **4,** wherein the phosphorothioate oligonucleotide is selected from the group comprising antisense oligonucleotides, siRNAs, shRNAs, ribozymes, aptamers, molecular decoys and RNA-DNA hybrid molecules, preferably said phosphorothioate oligonucleotide is an antisense oligonucleotide.

6. The composition according to any one of claims **1** to **5,** wherein the phosphorothioate oligonucleotide is an antisense oligonucleotide specific for IRS-1 (insulin receptor substrate-1), preferably the IRS-1 antisense phosphorothioate oligonucleotide is a sequence of at least 12 nucleotides, of SEQ ID NO: 1, more preferably the IRS-1 antisense phosphorothioate oligonucleotide is SEQ ID NO: 2 or a function-conservative derivative thereof.

7. The composition according to claim **6,** wherein function-conservative derivative of SEQ ID NO: 2 comprises from 9 to 50 nucleotides, has at least about 75% identity compared to SEQ ID NO: 2 and conserves the capacity of inhibiting IRS-1 expression as SEQ ID NO: 2, preferably said function-conservative derivative of SEQ ID NO: 2 is selected from SEQ ID NO: 3 to SEQ ID NO: 28.

8. The composition according to any one of claims **1** to **7,** wherein the phosphorothioate oligonucleotide is stable for at least 1 day at room temperature, and/or wherein the phosphorothioate oligonucleotide is stable for at least 10 minutes at 60°C.

9. The composition according to any one of claims **1** to **8,** wherein said composition is an emulsion comprising
- an aqueous phase, wherein the amount of the aqueous phase in the emulsion ranges from about 70 to 99% in weight to the total weight of the emulsion; and
- an oily phase, wherein the amount of the oily phase in the emulsion ranges from about 1 to 30% in weight to the total weight of the emulsion;
wherein the aqueous phase comprises:
- a phosphorothioate oligonucleotide, preferably in an amount ranging from about 0.01% to about 3% in weight to the total weight of the emulsion; or in an amount ranging from about 0.01% to about 3% in weight to the total weight of the aqueous phase of the emulsion, wherein said phosphorothioate oligonucleotide is preferably an IRS-1 antisense;
- an agent containing a thiol group, preferably in an amount ranging from about 0.5% to about 5% in weight to the total weight of the composition; or in an amount ranging from about 0.5% to about 5% in weight to the total weight of the aqueous phase of the emulsion of the invention ;
- at least one polymer of acrylic acid, preferably in an amount ranging from about 0.01% to about 0.1% in weight to the total weight of the emulsion;
- NaOH in an amount sufficient for buffering the emulsion at a pH ranging from about 6 to about 7, such as, for example, ranging from about 0.1 to about 0.5% in weight to the total weight of the emulsion; and
wherein the oily phase comprises:
- Medium Chain Triglycerides (MCT), preferably in an amount ranging from about 1 to about 20% in weight to the total weight of the emulsion;
- a mixture of glyceryl stearate and of PEG-75, preferably in an amount ranging from about 1% to about 10% in weight to the total weight of the emulsion; and
- cetyl alcohol, preferably in an amount ranging from about 0.5 to about 25% in weight to the total weight of the emulsion.

10. A pharmaceutical composition comprising a composition according to any one of claims **1** to **9** and at least one pharmaceutically acceptable excipient.

11. A medicament comprising a composition according to any one of claims **1** to **9.**

12. A method for obtaining a sterile composition comprising a phosphorothioate oligonucleotide and at least one fatty acid and/or at least one emulsifying agent, wherein said method comprises:
- mixing the components of the composition and an agent comprising a thiol group;
- autoclaving the composition resulting from the previous step, and
- adding the phosphorothioate oligonucleotide to the autoclaved composition, preferably under sterile conditions.

13. The method according to claim **12,** wherein the autoclaving step comprises heating the emulsion at a temperature ranging from about 100°C to about 130°C for a period ranging from about 10 to about 30 minutes, preferably at about 121°C for about 20 minutes.

14. A composition obtained by the method according to the method of claim **12** or claim **13.**

15. A composition according to any one of claims **1** to **9** or **14,** wherein the phosphorothioate oligonucleotide is an IRS-1 antisense, for treating an angiogenic disorder.
